# EUROPEAN PATENT APPLICATION

(11) **EP 4 693 334 A1**
(43) Date of publication of application: **11.02.2026**
(21) Application number: 24779013.2
(22) Date of filing: 28.02.2024
(51) Int. Cl.: G16H 50/50, G16H 50/20

(54) **LEARNING DEVICE, LEARNING METHOD, LEARNING PROGRAM, ESTIMATION DEVICE, ESTIMATION METHOD, AND ESTIMATION PROGRAM**

(30) Priority: 24.03.2023 JP 2023048623; 24.03.2023 JP 2023048624
(71) Applicant: NTT Docomo Business, Inc., Tokyo 100-8019 (JP); National University Corporation Chiba University, Chiba-shi, Chiba 263-8522 (JP)
(72) Inventor: AIZU, Takuma, Tokyo 100-8019 (JP); OU, Soka, Tokyo 100-8019 (JP); MORISHITA, Tomoyuki, Tokyo 100-8019 (JP); SAKURAI, Yoichi, Tokyo 100-8019 (JP); MISAWA, Sonoko, Chiba-shi, Chiba 260-8677 (JP); SUICHI, Tomoki, Chiba-shi, Chiba 260-8677 (JP); YOSHIMURA, Kensuke, Chiba-shi, Chiba 260-8677 (JP)
(74) Representative: Hoffmann Eitle
(86) International application number: PCT/JP2024/007374
(87) International publication number: WO 2024/202874

(57) **Abstract**

A server (20) includes: a first preprocessing unit (24) that performs predetermined preprocessing on inspection data and medical care data of a patient; and a first estimation unit (25) that uses at least the inspection data and medical care data of a patient who has developed a rare disease after the preprocessing as learning data, and that estimates an onset probability of an estimation target patient for each of a plurality of rare diseases, based on the inspection data and the medical care data of the estimation target patient after the preprocessing, using a first estimation model (26) that learns a relationship between the inspection data and the medical care data of the patient and a onset probability of a rare disease of the patient, and that estimates an onset probability for each of the plurality of rare diseases.

## Description

### [Technical Field]

The present invention relates to a learning device, a learning method, a learning program, an estimation device, an estimation method, and an estimation program.

### [Background Art]

An estimation device that estimates the onset of a disease using an estimation model using machine learning has been proposed.

### [Citation List]

### [Non Patent Citation]

Patent Literature 1: Japanese Laid-open Patent Publication No. 2019-016235 A

### [Summary of Invention]

### [Technical Problem]

Here, it is difficult for general doctors to make an appropriate judgment on rare diseases, and there are many patients who miss opportunities for early diagnosis and early treatment. Therefore, it is desired to construct an estimation model for estimating the onset probability of a rare disease.

The present invention has been made in view of the above, and an object thereof is to provide a learning device, a learning method, a learning program, an estimation device, an estimation method, and an estimation program capable of constructing an estimation model for estimating the onset of a rare disease.

### [Solution to Problem]

In order to solve the above-described problems and achieve the object, a learning device according to the present invention includes: a registration unit that registers at least inspection data and medical care data of a patient who has developed a rare disease from a plurality of medical institutions; a preprocessing unit that performs predetermined preprocessing on inspection data and medical care data of a patient; an estimation unit that estimates an onset probability of an estimation target patient for each of a plurality of rare diseases based on the inspection data and medical care data of the estimation target patient after the preprocessing by using an estimation model that estimates an onset probability for each of the plurality of rare diseases; and a learning unit that uses at least the inspection data and the medical care data of the patient who has developed a rare disease after the preprocessing as learning data, and causes the estimation model to learn a relationship between the inspection data and the medical care data of the patient and an onset probability of a rare disease of the patient.

An estimation device according to the present invention includes: a preprocessing unit that performs predetermined preprocessing on inspection data and medical care data of a patient; and an estimation unit that estimates an onset probability of an estimation target patient for each of a plurality of rare diseases based on inspection data and medical care data of an estimation target patient after the preprocessing using an estimation model that uses at least inspection data and medical care data of a patient who has developed a rare disease after the preprocessing as learning data and learns a relationship between inspection data and medical care data of a patient and an onset probability of a rare disease of the patient, the estimation model estimating an onset probability for each of the plurality of rare diseases.

### [Advantageous Effects of Invention]

According to the present invention, it is possible to construct an estimation model for estimating the onset of a rare disease.

### [Brief Description of Drawings]

FIG. 1 is a diagram illustrating an overview of a learning phase of an estimation model in a first embodiment.
FIG. 2 is a diagram illustrating an overview of an estimation phase using the estimation model in the first embodiment.
FIG. 3 is a block diagram illustrating an example of a configuration of a processing system according to the first embodiment.
FIG. 4 is a diagram schematically illustrating an example of a configuration of a registrant server illustrated in FIG. 3.
FIG. 5 is a diagram for describing processing of a data extraction unit illustrated in FIG. 4.
FIG. 6 is a diagram schematically illustrating an example of a configuration of a server illustrated in FIG. 3.
FIG. 7 is a diagram describing processing of a first preprocessing unit illustrated in FIG. 6.
FIG. 8 is a diagram describing processing of the first preprocessing unit illustrated in FIG. 6.
FIG. 9 is a diagram describing processing of the first preprocessing unit illustrated in FIG. 6.
FIG. 10 is a diagram schematically illustrating an example of a configuration of a request source terminal illustrated in FIG. 3.
FIG. 11 is a sequence diagram illustrating a processing procedure of learning processing according to the first embodiment.
FIG. 12 is a sequence diagram illustrating a processing procedure of estimation processing according to the first embodiment.
FIG. 13 is a diagram schematically illustrating an example of a configuration of a server according to a second embodiment.
FIG. 14 is a diagram describing processing of a second preprocessing unit illustrated in FIG. 13.
FIG. 15 is a diagram describing processing of the second preprocessing unit illustrated in FIG. 13.
FIG. 16 is a sequence diagram illustrating a processing procedure of learning processing according to the second embodiment.
FIG. 17 is a sequence diagram illustrating a processing procedure of estimation processing according to the second embodiment.
FIG. 18 is a diagram schematically illustrating an example of a configuration of a server according to a third embodiment.
FIG. 19 is a diagram describing processing of a third preprocessing unit illustrated in FIG. 18.
FIG. 20 is a diagram describing processing of the third preprocessing unit illustrated in FIG. 18.
FIG. 21 is a sequence diagram illustrating a processing procedure of learning processing according to the third embodiment.
FIG. 22 is a sequence diagram illustrating a processing procedure of estimation processing according to the third embodiment.
FIG. 23 is a diagram schematically illustrating an example of a configuration of a server according to a fourth embodiment.
FIG. 24 is a sequence diagram illustrating a processing procedure of learning processing according to the fourth embodiment.
FIG. 25 is a sequence diagram illustrating a processing procedure of preprocessing and learning processing illustrated in FIG. 24.
FIG. 26 is a sequence diagram illustrating a processing procedure of estimation processing according to the fourth embodiment.
FIG. 27 is a block diagram illustrating another example of the configuration of the processing system according to the first embodiment.
FIG. 28 is a diagram illustrating a computer that executes a program.

### [Embodiments for Carrying Out the Invention]

Hereinafter, embodiments of a learning device, a learning method, a learning program, an estimation device, an estimation method, and an estimation program according to the present application will be described in detail with reference to the drawings. Note that the learning device, the learning method, the learning program, the estimation device, the estimation method, and the estimation program according to the present application are not limited by the embodiment.

### (First embodiment)

First, a first embodiment will be described. In the first embodiment, a case will be described in which an estimation model for estimating the onset probability of an estimation target patient for each of a plurality of rare diseases is realized by secret calculation artificial intelligence (AI) capable of calculating data in an encrypted state.

### [Learning phase]

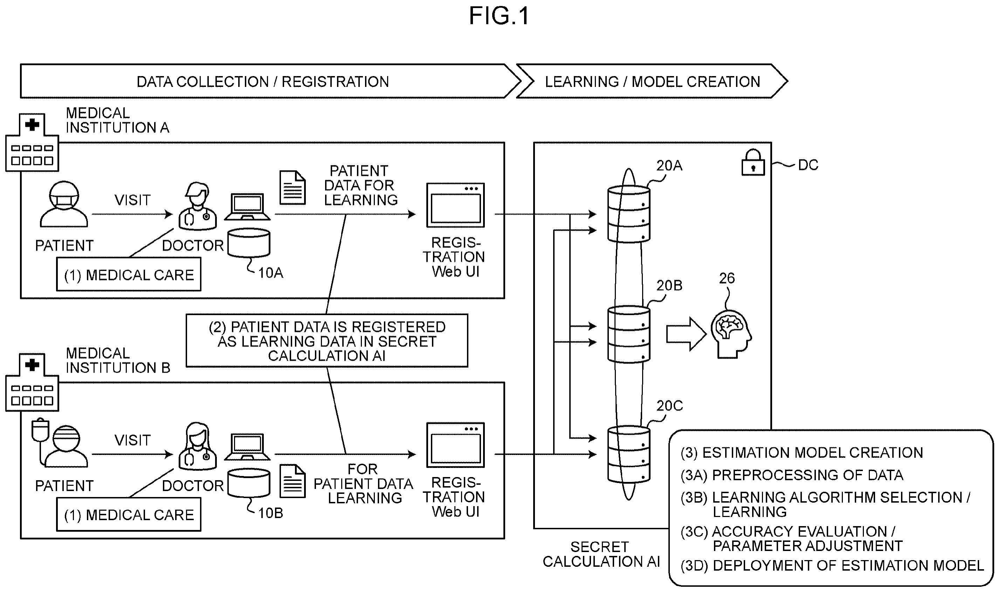

First, the learning phase of the estimation model will be described. FIG. 1 is a diagram illustrating an overview of a learning phase of an estimation model in a first embodiment.

As illustrated in FIG. 1, in medical institutions A and B, medical care for a visiting patient is performed ((1) of FIG. 1), and inspection data and medical care data (patient data) of a patient who has developed a rare disease are collected. The medical care data also includes rare disease information indicating a rare disease developed by this patient, a patient's medical history, and a family history.

Registrant servers 10A and 10B of the medical institutions A and B register patient data of a patient who has developed a rare disease as learning data in the secret calculation AI of the data center (DC) via a registration web user interface (UI) ((2) in FIG. 1). The learning data includes not only patient data of a patient who has developed a rare disease but also patient data of a patient who has developed a disease other than a rare disease. In the first embodiment, peripheral nerve diseases, specifically, chronic inflammatory demyelinating neuropathy, Guillain-Barré syndrome, POEMS syndrome, anti-MAG antibody-related neuropathy, Charcot-Marie-Tooth disease, and amyloidosis will be described as an example of rare diseases. Note that the exemplified rare diseases are examples, and are not limited to a disease of the cranial nervous system, and may be other diseases.

Then, in the DC, data is divided into fragments called a plurality of shares, and in a state where these shares are distributed and stored in a plurality of servers 20A to 20C, the plurality of servers 20A to 20C performs multi-party calculation for calculating and exchanging data among the plurality of servers 20A to 20C, thereby executing secret calculation (see, for example, Reference Literature 1.). Each share is meaningless data, and the original data cannot be restored with only one share and information is not leaked. However, when a certain number or more of shares are obtained, the original data can be restored.

Reference Literature 1: NIPPON TELEGRAPH AND TELEPHONE CORPORATION, Secret Calculation System and its Principle, (online), (searched on October 5, 2022), Internet <URL:https://www.rd.ntt/sc/project/data-security/NTT-himitsu-keisan.pdf>

In the first embodiment, a first estimation model 26 (for example, a machine learning model) for estimating the onset probability for each of the plurality of rare diseases is created by performing secret calculation among the plurality of servers 20A to 20C as the secret calculation AI ((3) in FIG. 1). The model parameters of the first estimation model 26 are distributed and stored in the plurality of servers 20A to 20C. The first estimation model 26 estimates the onset probability of a plurality of rare diseases for the encrypted patient data, and outputs an estimation result of the encrypted state.

First, the plurality of servers 20A to 20C performs predetermined preprocessing on the patient data for learning so that learning can be performed by the first estimation model 26 ((3A) in FIG. 1). The servers 20A to 20C select a learning algorithm, use at least patient data of a patient who has developed a rare disease after preprocessing as learning data, and cause the first estimation model 26 to learn (for example, machine learning) the relationship between the patient data of the patient and the onset probability of the rare disease of the patient ((3B) in FIG. 1). After performing the accuracy evaluation and the parameter adjustment ((3C) in FIG. 1), the servers 20A to 20C deploy the first estimation model 26 ((3D) in FIG. 1).

### (Estimation phase)

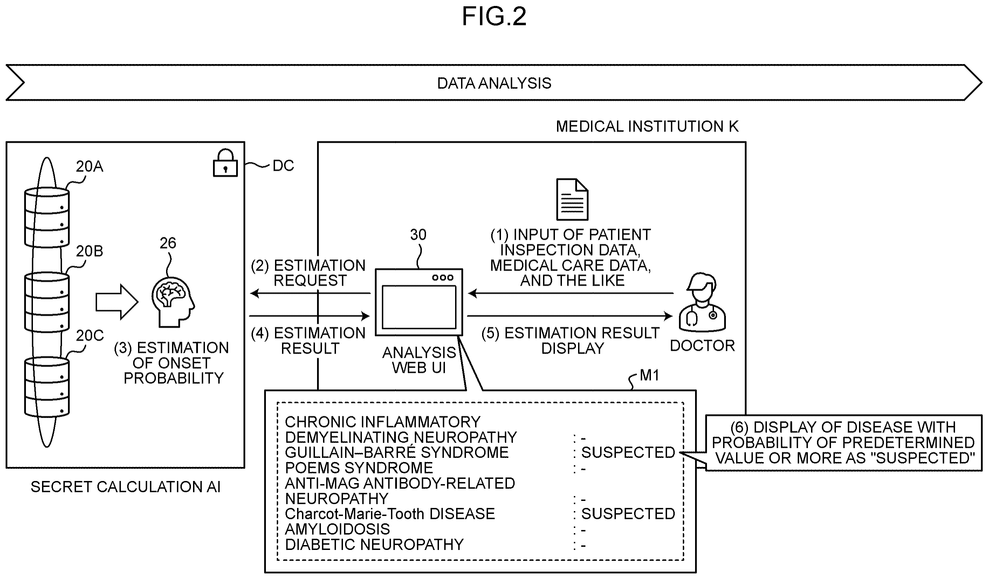

Next, the operation phase of the estimation model will be described with reference to FIG. 2. FIG. 2 is a diagram illustrating an overview of the estimation phase using the first estimation model 26.

As illustrated in FIG. 2, in the medical institution K as a request source, a doctor inputs inspection data, medical care data (patient data), and the like of an estimation target patient whose disease name cannot be determined to a request source terminal 30 ((1) in FIG. 2). The request source terminal 30 transmits, to the DC, patient data of an estimation target patient whose disease name cannot be determined and an estimation request of an onset probability of a rare disease of an estimation target patient whose disease name cannot be determined ((2) in FIG. 2).

In the DC, the servers 20A to 20C perform secret calculation to perform predetermined preprocessing on patient data of an estimation target patient whose disease name cannot be determined, and then estimate the onset probability of the rare disease of the estimation target patient using the first estimation model 26 ((3) in FIG. 2).

The request source terminal 30 receives the estimation results from the servers 20A to 20C ((4) in FIG. 2), decodes the received estimation results, and then displays the estimation results ((5) in FIG. 2). The request source terminal 30 displays "suspected" for a disease having an estimated onset probability of a predetermined value or more ((6) in FIG. 2).

For example, the request source terminal 30 displays a menu M1 in which a list of chronic inflammatory demyelinating neuropathy, Guillain-Barré syndrome, POEMS syndrome, anti-MAG antibody-related neuropathy, Charcot-Marie-Tooth disease, amyloidosis, and diabetic neuropathy is associated with a word "suspected" indicating that a disease having an estimated onset probability of a predetermined value or more is suspected of having onset. For example, a menu M1 indicates that an estimation target patient whose disease name cannot be determined is suspected of developing two diseases of Guillain-Barré syndrome and Charcot-Marie-Tooth disease.

### [Processing system]

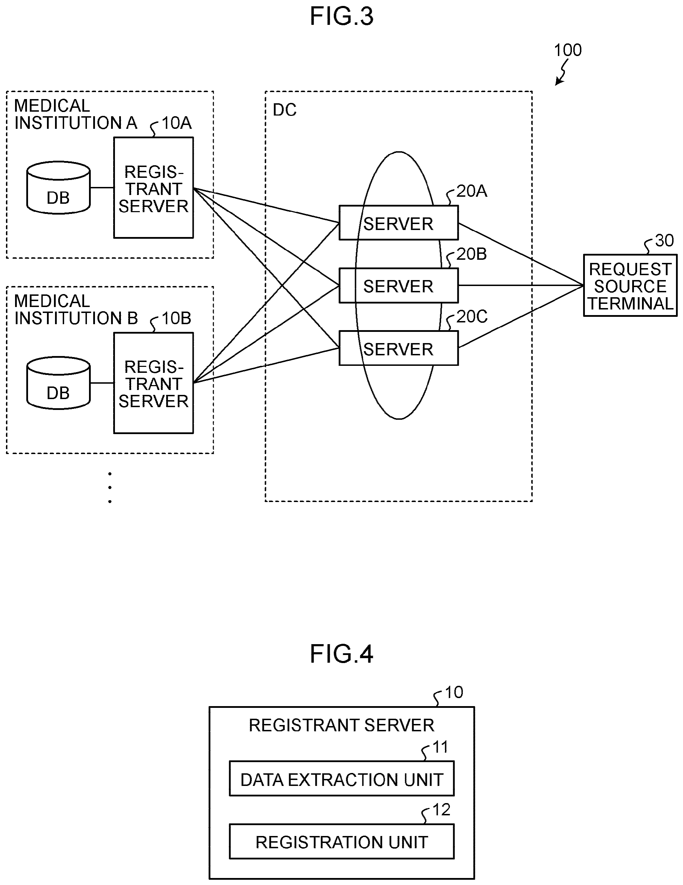

A processing system according to the first embodiment will be described. FIG. 3 is a block diagram illustrating an example of a configuration of a processing system according to the first embodiment.

A processing system 100 according to the first embodiment illustrated in FIG. 1 is a system that constructs an estimation model for estimating the onset probability of a plurality of rare diseases on secret calculation AI capable of calculating data in an encrypted state. In the first embodiment, as illustrated in FIG. 1, an example including the registrant servers 10A and 10B of medical institutions A and B that collect patient data of a rare disease for learning, the servers 20A, 20B, and 20C in the DC, and the request source terminal 30 that requests estimation of the onset probability of a rare disease of a target patient will be described.

Note that the configuration illustrated in FIG. 1 is merely an example, and a specific configuration and the number of devices are not particularly limited. In addition, the registrant servers 10A and 10B and the request source terminal 30 will be separately described for ease of description, but in actual operation, the function of the registrant servers 10A and 10B may be included in the request source terminal 30. In addition, in a case where the registrant servers 10A and 10B are collectively referred to, the registrant server 10 is used. In a case where the servers 20A, 20B, and 20C are collectively referred to, the server 20 is used.

In the medical institutions A and B, the registrant servers 10A and 10B create patient data obtained by extracting only data of a predetermined item from inspection data of a patient who has developed a rare disease or medical care data by a doctor and upload the patient data to the server 20 of the DC. The patient data is distributed and stored in the servers 20A to 20C of the DC in a state of being fragmented into shares. That is, each share obtained by fragmenting the patient data is distributed and stored in the servers 20A to 20C of the DC in an encrypted state.

The servers 20A to 20C of the DC learn an estimation model and perform estimation using the estimation model by performing multi-party calculation for calculating and exchanging data among the servers 20A to 20C.

In the learning phase, the servers 20A to 20C perform predetermined preprocessing on the patient data for learning registered by the registrant servers 10A and 10B, and then cause the first estimation model 26 to learn it.

In addition, in the estimation phase, when receiving patient data of an estimation target patient whose disease name cannot be determined and an estimation request of an onset probability of a rare disease of this patient from the request source terminal 30, the servers 20A to 20C perform predetermined preprocessing on the patient data of the estimation target patient. Then, the servers 20A to 20C estimate the onset probability of this patient for each of the plurality of rare diseases using the first estimation model 26 based on the patient data after the preprocessing, and transmit the estimation result to the request source terminal 30.

The request source terminal 30 decodes the received estimation result, and then displays "suspected" of onset of a disease for which the estimated onset probability is a predetermined value or more among a plurality of rare diseases.

### [Registrant server]

Next, a configuration of each device of the processing system 100 will be described. FIG. 4 is a diagram schematically illustrating an example of a configuration of the registrant server 10 illustrated in FIG. 3.

The registrant server 10 is implemented by a predetermined program being read by a computer and the like including a read only memory (ROM), a random access memory (RAM), a central processing unit (CPU), and the like and the CPU executing the predetermined program. In addition, the registrant server 10 has a communication interface that transmits and receives various types of information to and from another device connected via a network and the like. For example, the registrant server 10 includes a network interface card (NIC) and the like, and performs communication with another device via a telecommunication line such as a local area network (LAN) or the Internet. Then, the registrant server 10 includes an input device such as a touch panel, a voice input device, a keyboard and a mouse, and a display device such as a liquid crystal display, and inputs and outputs information. The registrant server 10 includes a data extraction unit 11 and a registration unit 12.

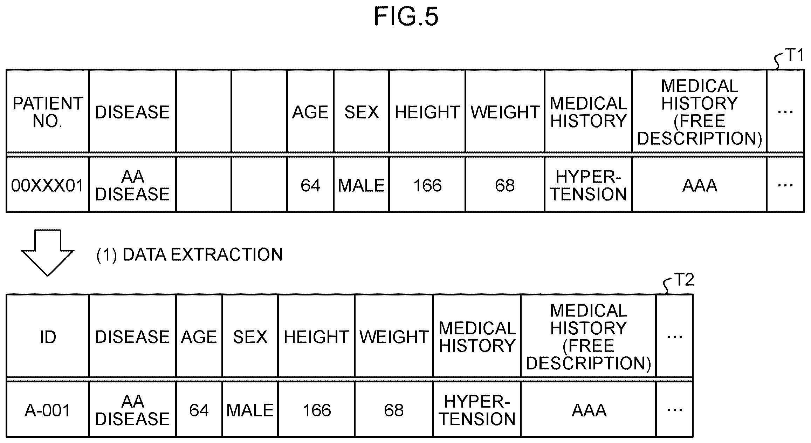

The data extraction unit 11 extracts patient data for learning registered in the servers 20A to 20C in a confidential distribution from inspection data and medical care data of a patient who has developed a rare disease, registered in a database (DB) of the medical institution A. FIG. 5 is a diagram for describing processing of the data extraction unit 11 illustrated in FIG. 4.

A table T1 is an example of a medical care result, and includes items such as patient number, patient name (not illustrated), disease (disease name),..., age (numerical value), sex, height (numerical value), weight (numerical value), medical history, and medical history (free description).

The data extraction unit 11 extracts items necessary for learning of the first estimation model 26 from the table T1. For example, the data extraction unit extracts a plurality of items (for example, 213 items) such as ID, disease (disease name), age (numerical value), sex, height (numerical value), weight (numerical value), medical history, medical history (free description), family history (similar disease), family history (disease name), main complaint, main complaint (free description), site of symptom, difference between right and left symptoms, onset age, onset mode, onset mode (own), and grip strength (number) from the table T1, and creates a table T2.

Note that the data extraction unit 11 extracts data of items necessary for learning of the first estimation model 26 from patient data accumulated by an electronic medical record system and the like. In addition, the data extraction unit 11 may extract data corresponding to an item necessary for learning of the first estimation model 26 by recognizing content described in a paper medical record by image recognition and the like.

The registration unit 12 makes a request to divide the patient data for learning (for example, the table T1) extracted by the data extraction unit 11 into a plurality of shares and distribute and register the divided shares to 20A to 20C, respectively. For example, the operator of the registrant server 10 performs selection of registration data and a distributed storage request of a share via a WebUI screen for the processing system 100 displayed on a web browser.

### (Server)

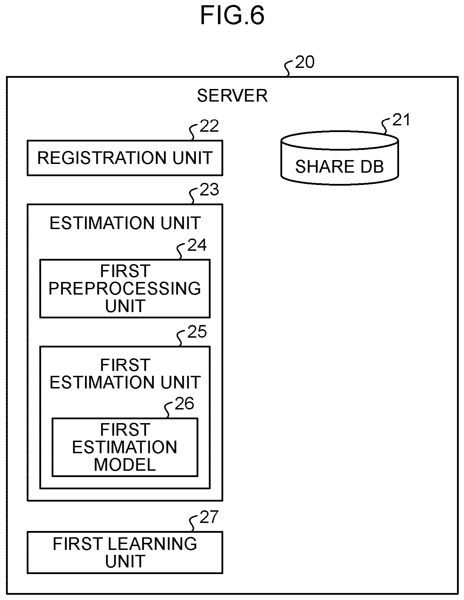

Next, a configuration of the server 20 will be described. FIG. 6 is a diagram schematically illustrating an example of a configuration of the server 20 illustrated in FIG. 3.

The server 20 is implemented by a predetermined program being read by a computer and the like including a read only memory (ROM), a random access memory (RAM), a CPU, and the like and the CPU executing the predetermined program. In addition, the server 20 has a communication interface that transmits and receives various types of information to and from another device connected via a network and the like. For example, the server 20 includes an NIC and the like, and performs communication with another device via a telecommunication line such as a LAN or the Internet. Then, the server 20 includes an input device such as a touch panel, a voice input device, a keyboard and a mouse, and a display device such as a liquid crystal display, and inputs and outputs information. The server 20 includes a share DB 21, a registration unit 22, an estimation unit 23, and a first learning unit 27.

The share DB 21 stores, for example, a share requested to be registered by the registrant servers 10A and 10B. The share is, for example, patient data for learning.

The registration unit 22 registers the share requested to be registered from the registrant servers 10A and 10B in the share DB 21. By receiving the registration request from the registrant servers 10A and 10B, the registration unit 22 acquires and registers inspection data and medical care data of a patient who has developed a rare disease and inspection data and medical care data of a patient who has developed a disease other than a rare disease from the plurality of medical institutions A and B.

The estimation unit 23 estimates the onset probability of the estimation target patient for each of a plurality of rare diseases based on patient data of the estimation target patient whose disease name cannot be determined. The estimation unit 23 performs estimation processing on secret calculation without restoring data. The estimation unit 23 includes a first preprocessing unit 24 and a first estimation unit 25 having the first estimation model 26.

The first preprocessing unit 24 performs predetermined preprocessing on inspection data and medical care data (patient data) of a patient. The first preprocessing unit 24 performs first preprocessing of setting items related to patient background, medical history, clinical examination finding, or inspection finding in stages according to the granularity, and converting data of each item into corresponding categorical variables, for patient data.

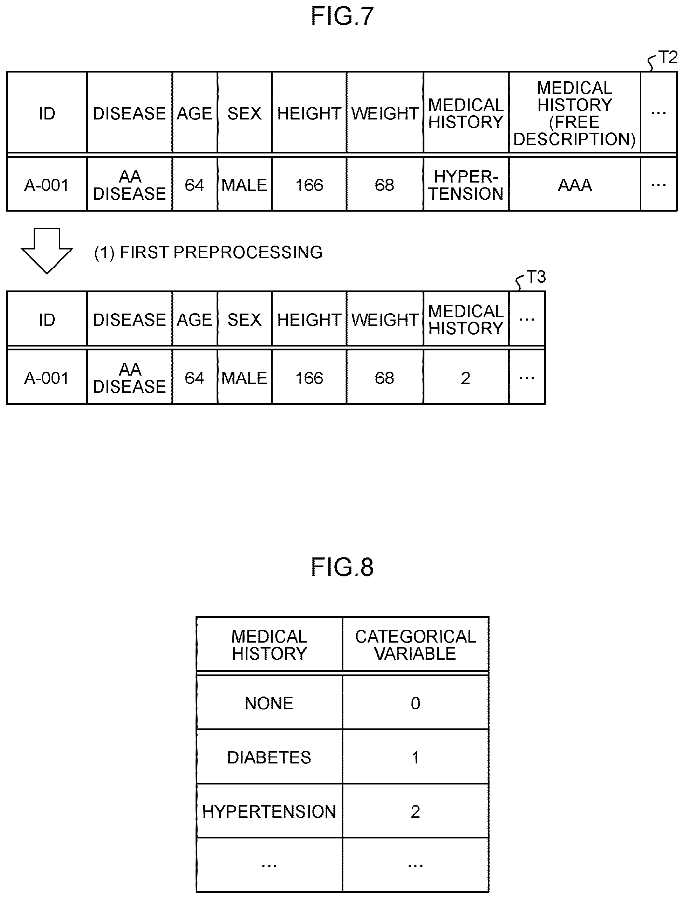

FIGS. 7 and 8 are diagrams describing processing of the first preprocessing unit 24 illustrated in FIG. 6. The first preprocessing unit 24 performs first preprocessing for converting data of items other than numerical values such as the disease name of the patient and the disease name, age, height, and the like of the family history into corresponding categorical variables on the table T2 registered from the registrant server 10 ((1) in FIG. 7).

For example, the first preprocessing unit 24 converts the medical history into a numerical value according to the correspondence table illustrated in FIG. 8. The first preprocessing unit 24 converts the data of the item of the medical history into a categorical variable "0" in a case where there is no medical history, converts the data into a categorical variable "1" in a case where the medical history is diabetes, and converts the data into a categorical variable "2" in a case where the medical history is hypertension. In addition, the first preprocessing unit 24 deletes the free description item. As a result, the first preprocessing unit 24 generates a table T3 in which the item "hypertension" of the medical history is converted into the categorical variable "2", for example. In this manner, the first preprocessing unit 24 converts the data corresponding to each item into a numerical value or a categorical variable.

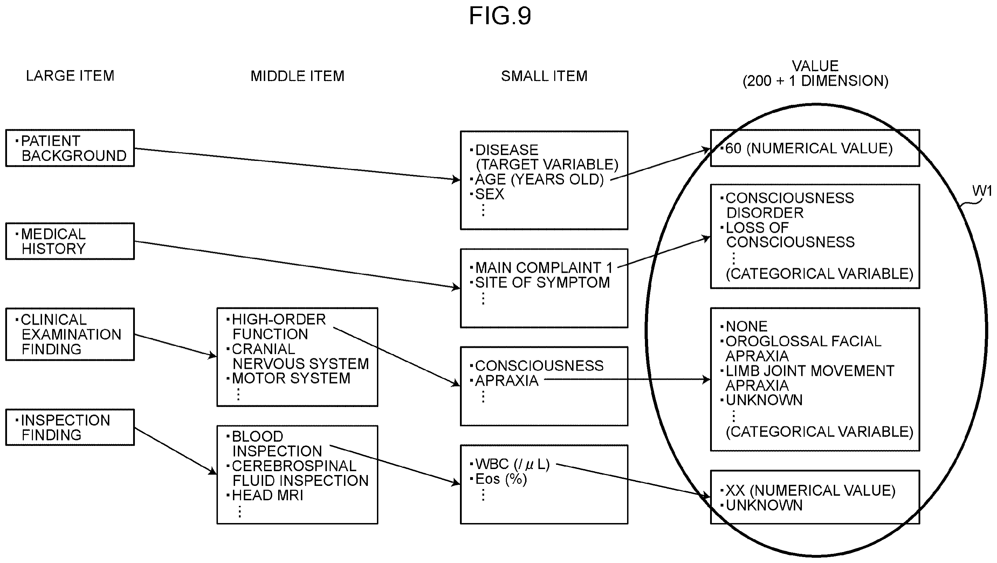

Then, the first preprocessing unit 24 sets items regarding patient background, medical history, clinical examination finding, or inspection finding in stages according to the granularity with respect to the patient data. FIG. 9 is a diagram describing processing of the first preprocessing unit 24 illustrated in FIG. 6.

For example, as illustrated in FIG. 9, the first preprocessing unit 24 associates items with the patient background as a large item and disease (objective variable), age (years old), and sex as small items.

The first preprocessing unit 24 associates the medical history as a large item and the main complaint 1 and the site of symptom as small items. The first preprocessing unit 24 assigns each categorical variable indicating consciousness disorder and loss of consciousness to the item of the main complaint 1.

The first preprocessing unit 24 associates the clinical examination finding as a large item and items of a high-order function, a cranial nervous system, and a motor system as middle items. Then, the first preprocessing unit 24 associates items of consciousness and apraxia as small items of high-order functions. In the item of apraxia, each categorical variable indicating none, oroglossal facial apraxia, limb joint movement apraxia, and unknown is assigned.

The first preprocessing unit 24 associates the inspection finding as a large item, and items of a blood inspection, a cerebrospinal fluid inspection, and a head magnetic resonance imaging (MRI) as middle items. Then, the first preprocessing unit 24 associates WBC (/µL) and Eos (%) as small items of the blood inspection, and assigns numerical values to the small items.

As described above, the first preprocessing unit 24 performs the first preprocessing on the patient data so as to be data that can be input to the first estimation model 26.

The first estimation unit 25 performs first estimation processing of estimating the onset probability of the estimation target patient for each of a plurality of rare diseases based on patient data of the estimation target patient whose disease name cannot be determined after the first preprocessing using the first estimation model 26.

The first estimation model 26 is a model that estimates the onset probability for each of a plurality of rare diseases. When the patient data after the first preprocessing is input, the first estimation model 26 outputs data in which a plurality of rare diseases is associated with the onset probability of each rare disease of the patient. The model parameters of the first estimation model 26 are distributed and stored in the plurality of servers 20A to 20C. Each first estimation unit 25 of the servers 20A to 20C performs multi-party calculation between the servers 20A to 20C to estimate the onset probability of each rare disease of this patient based on patient data that has been encrypted and subjected to the first preprocessing.

The first learning unit 27 uses the inspection data and the medical care data of the patient who has developed a rare disease and the inspection data and the medical care data of the patient who has developed a disease other than a rare disease after the first preprocessing as learning data, and causes the first estimation model 26 to learn a relationship between the inspection data and the medical care data of the patient and the onset probability of the rare disease of the patient.

The first learning unit 27 inputs inspection data and medical care data (excluding rare disease information), which are learning data after the first preprocessing, to the first estimation model 26, and performs first parameter update processing of updating a parameter of the first estimation model 26 so that the onset probability of each rare disease estimated by the first estimation model 26 approaches the rare disease actually diagnosed. For example, the first learning unit 27 executes learning of the first estimation model 26 until a predetermined end condition is satisfied. Note that the end condition is, for example, that the processing has been repeated a certain number of times, that the parameter update amount has converged, and the like.

### [Request source terminal]

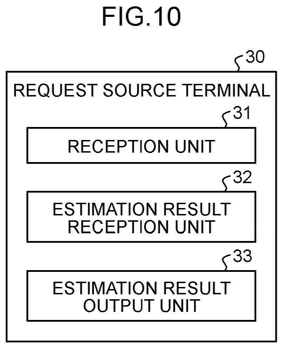

Next, a configuration of the request source terminal 30 will be described. FIG. 10 is a diagram schematically illustrating an example of a configuration of the request source terminal 30 illustrated in FIG. 3.

The request source terminal 30 is implemented by a predetermined program being read by a computer and the like including a ROM, a RAM, a CPU, and the like and the CPU executing the predetermined program. In addition, the request source terminal 30 has a communication interface that transmits and receives various types of information to and from another device connected via a network and the like. For example, the request source terminal 30 includes an NIC and the like, and performs communication with another device via a telecommunication line such as a LAN or the Internet. Then, the request source terminal 30 includes an input device such as a touch panel, a voice input device, a keyboard and a mouse, and a display device such as a liquid crystal display, and inputs and outputs information. The request source terminal 30 includes a reception unit 31, an estimation result reception unit 32, and an estimation result output unit 33.

In accordance with an operation of an operator (doctor and the like) of the request source terminal 30, the reception unit 31 receives input of inspection data and medical care data of a rare disease estimation target patient whose disease name cannot be determined. The reception unit 31 extracts data of an item necessary for estimation of the first estimation model 26 from the inspection data and the medical care data of the rare disease estimation target patient, and transmits a request for estimation of the onset probability of the rare disease of the estimation target patient to the DC together with the extracted patient data.

The estimation result reception unit 32 receives the estimation result in the encrypted state from each of the servers 20A to 20C.

The estimation result output unit 33 restores each received calculation result and outputs the onset probability for each of a plurality of rare diseases for rare disease estimation target patient whose disease name cannot be determined. For example, the estimation result output unit 33 displays a list of a plurality of rare diseases, and displays "suspected" for a disease having an estimated onset probability of a predetermined value or more.

### [Learning processing]

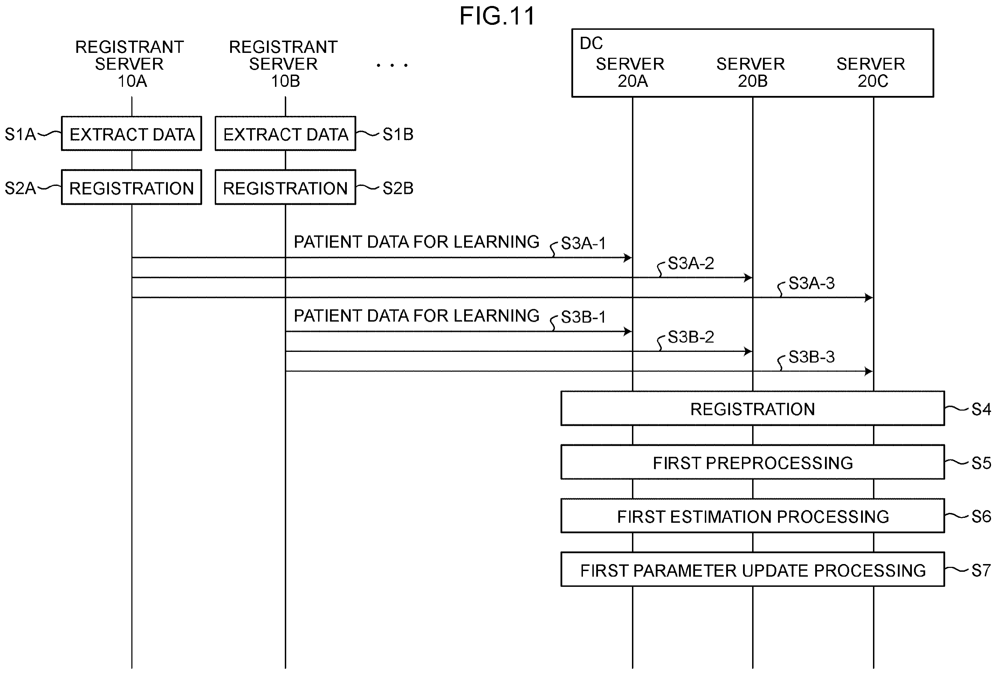

Next, learning processing according to the first embodiment will be described. FIG. 11 is a sequence diagram illustrating a processing procedure of learning processing according to the first embodiment.

As illustrated in FIG. 11, the registrant servers 10A and 10B extract patient data for learning from inspection data and medical care data of a patient who has developed a rare disease (steps S1A and S1B), divide the patient data into a plurality of shares, and distribute and register the divided shares in the servers 20A to 20C, respectively (steps S2A, S2B, S3A-1 to S3A-3, S3B-1 to S3B-3, S4).

After performing the first preprocessing on the patient data for learning (step S5), the servers 20A to 20C perform the first estimation processing of estimating the onset probability of this patient for each of the plurality of rare diseases based on the patient data for learning after the first preprocessing (step S6).

The servers 20A to 20C perform the first parameter update processing of updating the parameters of the first estimation model 26 so that the onset probability of each rare disease estimated by the first estimation model 26 approaches the rare disease actually diagnosed (step S7). The servers 20A to 20C repeatedly execute steps S5 to S7 until a predetermined end condition is satisfied. The servers 20A to 20C execute steps S4 to S7 on the secret calculation AI.

### (Estimation processing)

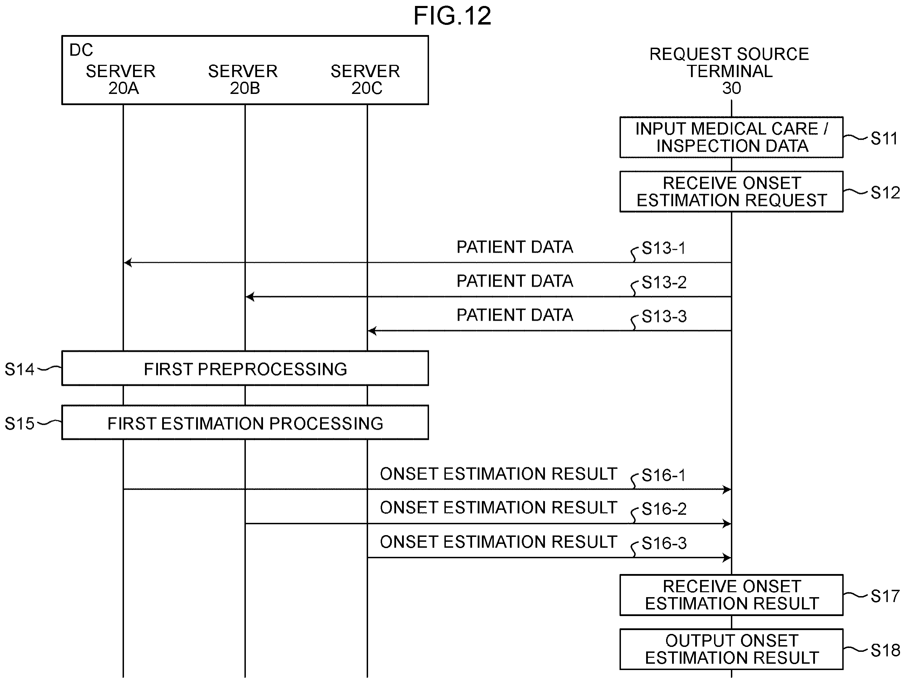

Next, estimation processing according to the first embodiment will be described. FIG. 12 is a sequence diagram illustrating a processing procedure of estimation processing according to the first embodiment.

As illustrated in FIG. 12, the request source terminal 30 receives inputs of inspection data and medical care data (patient data) of a rare disease estimation target patient whose disease name cannot be determined, and an estimation request of the onset probability of the rare disease of the estimation target patient (steps S11 and S12). The request source terminal 30 extracts data of an item necessary for estimation of the first estimation model 26 from the inspection data and the medical care data of the rare disease estimation target patient, and transmits a rare disease onset probability estimation request to the DC servers 20A to 20C together with the extracted patient data (steps S13-1 to S13-3).

After performing the first preprocessing on the estimation target patient data (step S14), the servers 20A to 20C perform the first estimation processing of estimating the onset probability of this patient for each of the plurality of rare diseases based on the estimation target patient data after the first preprocessing (step S15). The servers 20A to 20C execute steps S14 and S15 on the secret calculation AI. The servers 20A to 20C transmit the estimation result of the onset probability of a rare disease of the estimation target patient to the request source terminal 30 (steps S16-1 to S16-3).

The request source terminal 30 receives the estimation result (step S17), decodes the received estimation result, and then displays the estimation result of the onset probability of the rare disease of the estimation target patient (step S18). The request source terminal 30 displays "suspected" for a disease having an estimated onset probability of a predetermined value or more among a plurality of rare diseases.

### (Effect of first embodiment)

As described above, in the first embodiment, the servers 20A to 20C create and use the first estimation model 26 for estimating the onset probability for each of a plurality of rare diseases, whereby diagnosis of a rare disease with a small number of cases can be supported, and promotion of early diagnosis and early treatment of a rare disease is realized.

In addition, the servers 20A to 20C learn patient data while being encrypted on the secret calculation AI. Therefore, according to the first embodiment, patient data including personal information can be safely handled, and patient data can be collected from a plurality of medical institutions even in a rare disease with a small number of cases. Therefore, according to the first embodiment, the amount of patient data necessary for learning of the first estimation model 26 can be collected, and appropriate learning of the first estimation model 26 becomes possible.

In addition, in the first embodiment, the onset probability of a rare disease of an estimation target patient whose disease name cannot be determined is estimated while being encrypted on the secret calculation AI. Therefore, according to the first embodiment, it is possible to estimate the onset probability of a rare disease with a small number of cases while safely handling patient data including personal information. In addition, in the first embodiment, the request source terminal 30 displays a plurality of rare diseases having a possibility of onset, so that it is possible to support a doctor to perform diagnosis in a wide field of view.

### (Second embodiment)

Next, a second embodiment will be described. In the second embodiment, the categorical variable part of the item having the finest granularity among the items of the inspection data and the medical care data is labeled according to the meaning of the data corresponding to the categorical variable, and second preprocessing of compressing with the same label is performed, thereby improving the efficiency of learning (for example, machine learning).

The processing system according to the second embodiment includes a server 220 instead of the server 20 according to the first embodiment. Note that, also in the second embodiment, the DC includes a plurality of servers 220 (220A to 220C to be described later), and the plurality of servers 220 executes various types of processing to be described below by distributing and storing various types of data in a state of being fragmented into shares and performing multi-party calculation among the plurality of servers 220.

### (Server)

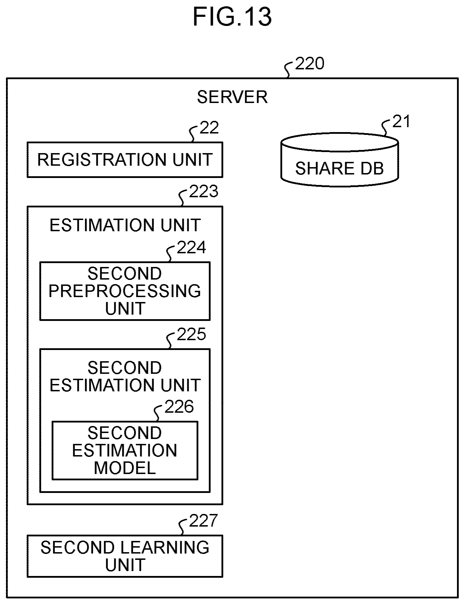

Next, a configuration of the server 220 will be described. FIG. 13 is a diagram schematically illustrating an example of a configuration of the server 220 according to the second embodiment. The server 220 includes an estimation unit 223 and a second learning unit 227 instead of the estimation unit 23 and the first learning unit 27 of the server 20 illustrated in FIG. 6.

The estimation unit 223 includes a second preprocessing unit 224 and a second estimation unit 225 having a second estimation model 226 (for example, a machine learning model).

The second preprocessing unit 224 performs second preprocessing on inspection data and medical care data (patient data) of a patient. The second preprocessing unit 224 performs, as second preprocessing, sets items related to patient background, medical history, clinical examination finding, or inspection finding in stages according to the granularity, and converts data of each item into corresponding categorical variables, for patient data. Then, as the second preprocessing, the second preprocessing unit 224 labels the categorical variable part of the item having the finest granularity according to the meaning of the data corresponding to the categorical variable, and compresses the categorical variable part with the same label.

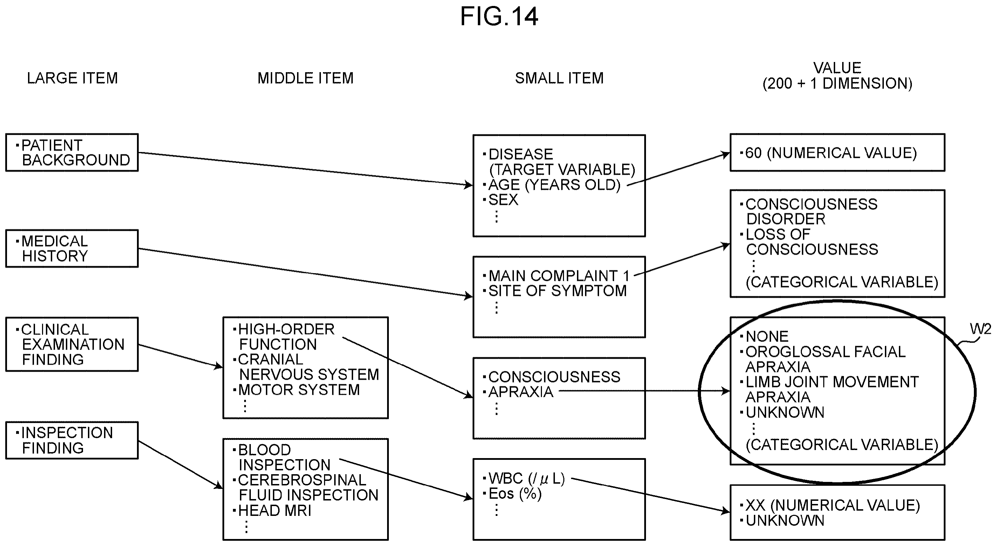

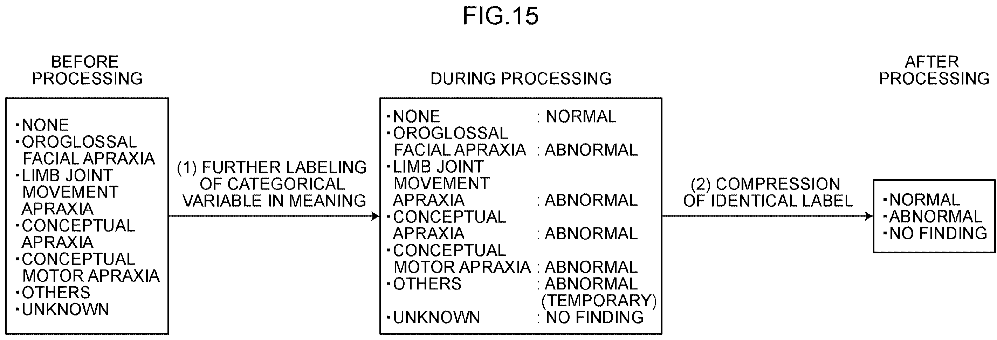

FIGS. 14 and 15 are diagrams describing processing of the second preprocessing unit 224 illustrated in FIG. 13. As illustrated in FIG. 14, the second preprocessing unit 224 compresses, for example, a categorical variable part (frame W2) of "apraxia" which is a small item of clinical examination finding according to a meaning of data corresponding to the categorical variable.

As illustrated in FIG. 15, the second preprocessing unit 224 further labels the categorical variables corresponding to "none", "oroglossal facial apraxia", "limb joint movement apraxia", "conceptual apraxia", "conceptual motor apraxia", " others", and "unknown" of the small item "apraxia" with the meaning of the data corresponding to the categorical variables ((1) in FIG. 15).

Specifically, the second preprocessing unit 224 assigns a "normal" label to a categorical variable corresponding to "none" among the categorical variables. The second preprocessing unit 224 assigns an "abnormal" label to the categorical variables corresponding to "oroglossal facial apraxia", "limb joint movement apraxia", "conceptual apraxia", and "conceptual motor apraxia". The second preprocessing unit 224 assigns an "abnormal (provisional)" label to a categorical variable corresponding to "others", and assigns a "no finding" label to a categorical variable corresponding to "unknown".

Then, the second preprocessing unit 224 compresses the same label ((2) in FIG. 15). For example, in the example of FIG. 15, since there are three types of labels "normal", "abnormal", and "no finding" assigned to each categorical variable of the small item "apraxia", the second preprocessing unit 224 compresses the categorical variables to "normal", "abnormal", and "no finding" for the small item "apraxia". Since the information amount (number of dimensions) is reduced by the type of the categorical variable by the second preprocessing, it is possible to improve the learning time of the second estimation model 226 (described later) and reduce the data amount necessary for learning.

The second estimation unit 225 performs second estimation processing of estimating the onset probability of the estimation target patient for each of a plurality of rare diseases based on patient data of the estimation target patient whose disease name cannot be determined after the second preprocessing using the second estimation model 226.

The second estimation model 226 is a model that estimates the onset probability for each of a plurality of rare diseases. When the patient data after the second preprocessing is input, the second estimation model 226 outputs data in which a plurality of rare diseases is associated with the onset probability of each rare disease of the patient. The model parameters of the second estimation model 226 are distributed and stored in the plurality of servers 220. Each second estimation unit 225 of the servers 220 performs multi-party calculation between the servers 220 to estimate the onset probability of each rare disease of this patient based on patient data that has been encrypted and subjected to the second preprocessing.

The second learning unit 227 uses the inspection data and the medical care data of the patient who has developed a rare disease and the inspection data and the medical care data of the patient who has developed a disease other than a rare disease after the second preprocessing as learning data, and causes the second estimation model 226 to learn a relationship between the inspection data and the medical care data of the patient and the onset probability of the rare disease of the patient.

The second learning unit 227 inputs inspection data and medical care data (excluding rare disease information), which are learning data after the second preprocessing, to the second estimation model 226, and performs second parameter update processing of updating a parameter of the second estimation model 226 so that the onset probability of each rare disease estimated by the second estimation model 226 approaches the rare disease actually diagnosed.

### [Learning processing]

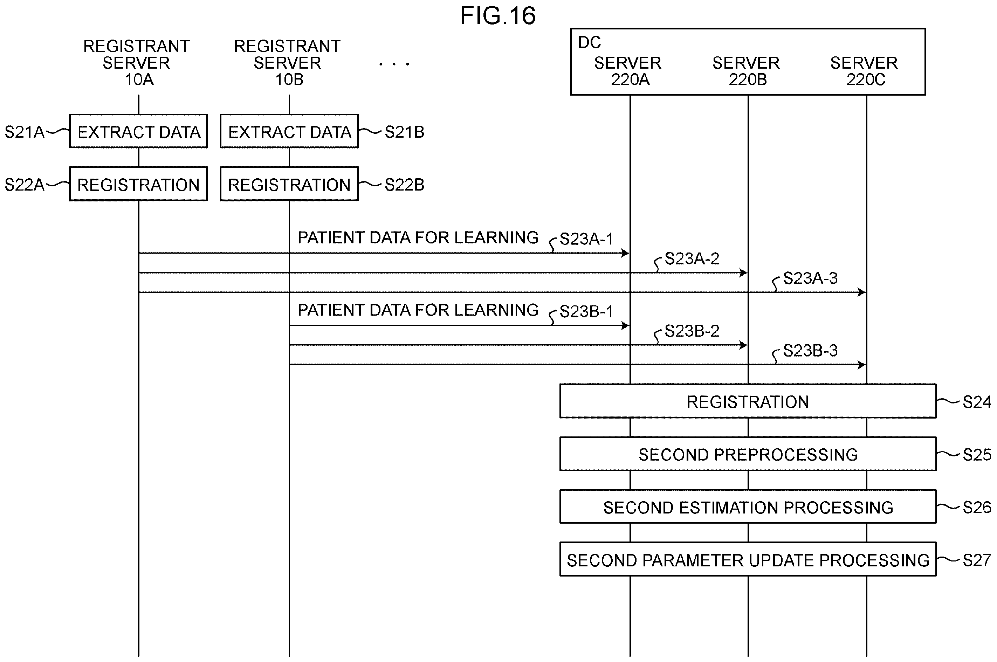

Next, learning processing according to the second embodiment will be described. FIG. 16 is a sequence diagram illustrating a processing procedure of learning processing according to the second embodiment.

Steps S21A to S24 illustrated in FIG. 16 are the same processing as steps S1A to S4 illustrated in FIG. 11.

After performing the second preprocessing on the patient data for learning (step S25), the servers 220A to 220C perform the second estimation processing of estimating the onset probability of this patient for each of the plurality of rare diseases based on the patient data for learning after the second preprocessing (step S26).

The servers 220A to 220C perform the second parameter update processing of updating the parameters of the second estimation model 226 so that the onset probability of each rare disease estimated by the second estimation model 226 approaches the rare disease actually diagnosed (step S27). The servers 220A to 220C repeatedly execute steps S25 to S27 until a predetermined end condition is satisfied. The servers 220A to 220C execute steps S24 to S27 on the secret calculation AI.

### (Estimation processing)

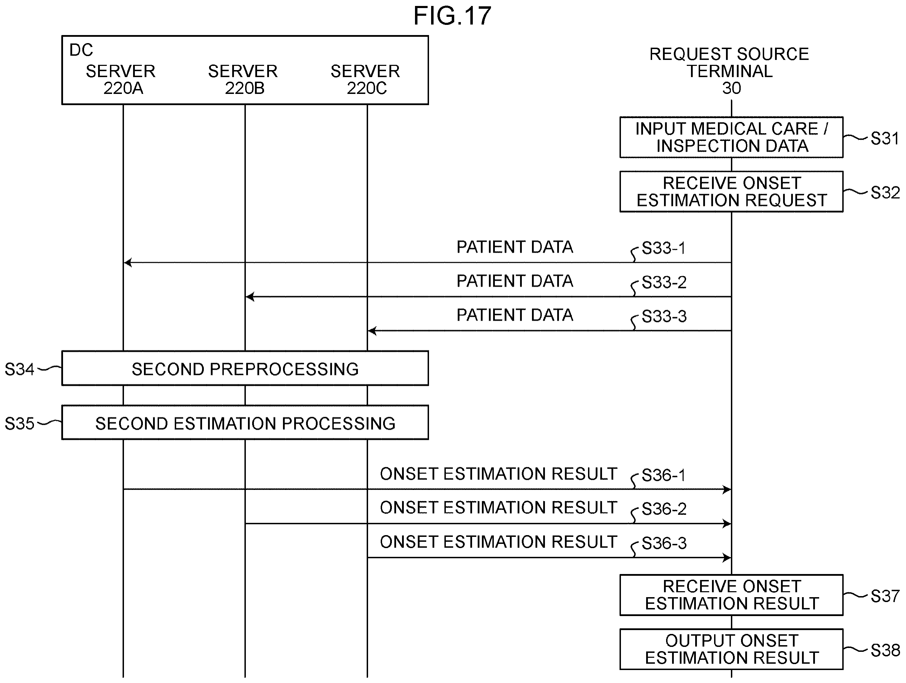

Next, estimation processing according to the second embodiment will be described. FIG. 17 is a sequence diagram illustrating a processing procedure of estimation processing according to the second embodiment.

Steps S31 to S33-3 illustrated in FIG. 17 are the same processing as steps S11 to S13-3 illustrated in FIG. 12.

After performing the second preprocessing on the data of estimation target patient whose disease name cannot be determined (step S34), the servers 220A to 220C perform the second estimation processing of estimating the onset probability of this patient for each of the plurality of rare diseases based on the estimation target patient data after the second preprocessing (step S35). The servers 220A to 220C execute steps S34 and S35 on the secret calculation AI. The servers 220A to 220C transmit the estimation result of the onset probability of a rare disease of the estimation target patient to the request source terminal 30 (steps S36-1 to S36-3). Steps S37 and S38 illustrated in FIG. 17 are the same processing as steps S17 and S18 illustrated in FIG. 12.

### (Effect of second embodiment)

In the second embodiment, the same effects as those of the first embodiment are obtained, and the information amount (number of dimensions) is reduced by the type of the categorical variable of the small item of the patient data by the second preprocessing, so that it is possible to improve the learning time of the second estimation model 226 (described later) and reduce the data amount necessary for learning.

Then, in the second embodiment, as in the first embodiment, by displaying a plurality of rare diseases having a possibility of onset, it is supported that a doctor can narrow down diseases from a plurality of rare diseases that are candidates based on an inspection and the like. In other words, an object of the second embodiment is to provide a doctor with awareness of the possibility that a patient has a rare disease.

Therefore, it can be said that the above object can be achieved if a plurality of candidate rare diseases is presented and the correct diseases are included therein. That is, in order to achieve the above object, it can be said that it should be emphasized that a correct rare disease is not omitted from a candidate rather than estimating one correct disease in a pinpoint manner. Therefore, in the second embodiment, even if the second preprocessing in which a disease in which another abnormality is observed in the same small item cannot be distinguished is performed, it is possible to reduce omission of a correct rare disease from a candidate and support diagnosis by a doctor by estimating the onset probability for a plurality of rare diseases.

### (Third embodiment)

Next, a third embodiment will be described. In the third embodiment, the categorical variable part of the first item having the finest granularity among the items of the inspection data and the medical care data is labeled according to the meaning of the data corresponding to the categorical variable, and compressed with the same label, and the categorical variable part of the second item having coarser granularity than the first item is compressed by performing the third preprocessing, thereby further improving the efficiency of learning (for example, machine learning).

The processing system according to the third embodiment includes a server 320 instead of the server 220 according to the second embodiment. Note that, also in the third embodiment, the DC includes a plurality of servers 320 (320A to 320C to be described later), and the plurality of servers 320 executes various types of processing to be described below by distributing and storing various types of data in a state of being fragmented into shares and performing multi-party calculation among the plurality of servers 320.

### (Server)

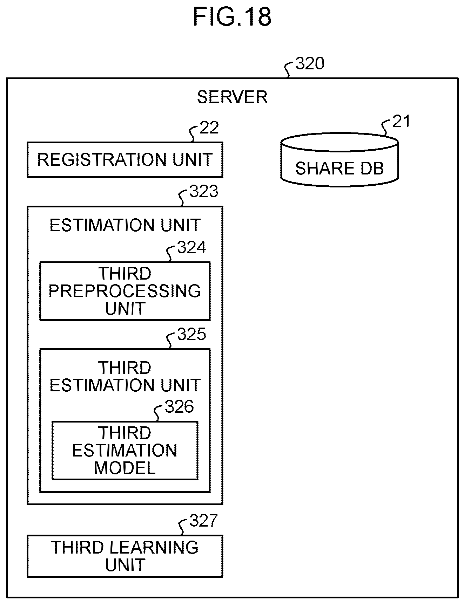

Next, a configuration of the server 320 will be described. FIG. 18 is a diagram schematically illustrating an example of a configuration of the server 320 according to the third embodiment. The server 320 includes an estimation unit 323 and a third learning unit 327 instead of the estimation unit 223 and the second learning unit 227 of the server 220 illustrated in FIG. 13.

The estimation unit 323 includes a third preprocessing unit 324 and a third estimation unit 325 having a third estimation model 326 (for example, a machine learning model).

The third preprocessing unit 324 performs predetermined preprocessing on inspection data and medical care data (patient data) of a patient. The third preprocessing unit 324 performs third preprocessing.

The third preprocessing unit 324 performs, as third preprocessing, sets items related to patient background, medical history, clinical examination finding, or inspection finding in stages according to the granularity, and converts data of each item into corresponding categorical variables, for patient data. Then, as the third preprocessing, the third preprocessing unit 324 labels the categorical variable part of the first item having the finest granularity according to the meaning of the data corresponding to the categorical variable, and compresses the categorical variable part with the same label. At the same time, the third preprocessing unit 324 performs, as the third preprocessing, third preprocessing of expressing the second item having a coarser granularity than the first item by the number of counts of each compressed label of the first item belonging to the second item.

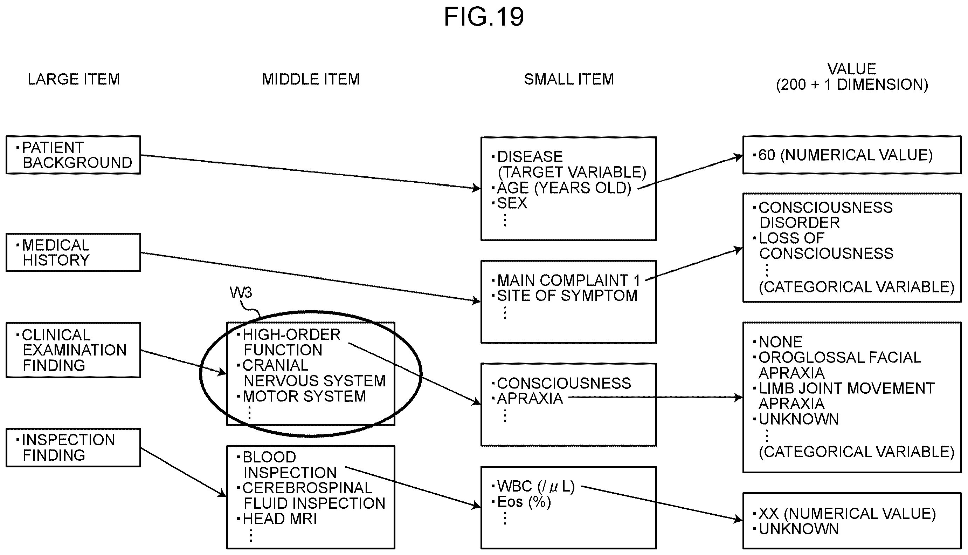

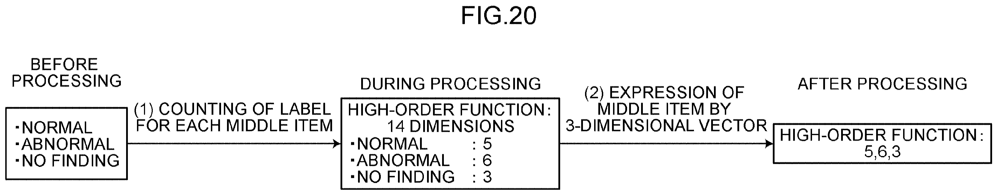

FIGS. 19 and 20 are diagrams describing processing of the third preprocessing unit 324 illustrated in FIG. 18. As illustrated in FIGS. 14 and 15 described above, the third preprocessing unit 324 labels the categorical variable part of the small item having the finest granularity according to the meaning of the data corresponding to the categorical variable, and compresses the categorical variable part with the same label.

Then, as illustrated in FIG. 19, the third preprocessing unit 324 further compresses information in units of middle items having coarser granularity than the small items, for example. For example, the third preprocessing unit 324 compresses information of the middle items "high-order function", "cranial nervous system", and "motor system" (frame W3) of the large item "clinical examination finding".

Specifically, the middle item "high-order function" in "clinical examination finding" will be described as an example. For example, as illustrated in FIG. 20, the third preprocessing unit 324 counts each compressed label of all the small items belonging to the middle item "high-order function" ((1) in FIG. 20). In this case, all the small items belonging to the middle item "high-order function" are compressed into three types of labels of "normal", "abnormal", and "no finding".

The third preprocessing unit 324 counts each compressed label "normal", "abnormal", and "no finding" of all the small items belonging to the middle item "high-order function". As a result, in the middle item "high-order function" (for example, 14 dimensions), the "normal" label is counted as 5, the "abnormal" label is counted as 6, and the "no finding" label is counted as 3.

Then, the third preprocessing unit 324 expresses the middle item "high-order function" by the number of counts "5, 6, 3" of the compressed labels "normal", "abnormal", and "no finding" of all the small items belonging to the middle item "high-order function" ((2) in FIG. 20). By the third preprocessing, the middle item "high-order function" expressed in 14 dimensions can be expressed by a three-dimensional vector, so that the amount of information (number of dimensions) is reduced, and the learning time of the third estimation model 326 (described later) can be improved and the amount of data requested for learning can be reduced. In this case, even if the middle items are all unknown data and the like, the input is not sparse.

The third estimation unit 325 performs third estimation processing of estimating the onset probability of the estimation target patient for each of a plurality of rare diseases based on patient data of the estimation target patient whose disease name cannot be determined after the third preprocessing using the third estimation model 326.

The third estimation model 326 is a model that estimates the onset probability for each of a plurality of rare diseases. When the patient data after the third preprocessing is input, the third estimation model 326 outputs data in which a plurality of rare diseases is associated with the onset probability of each rare disease of the patient. The model parameters of the third estimation model 326 are distributed and stored in the plurality of servers 320. Each third estimation unit 325 of the servers 320 performs multi-party calculation between the servers 320 to estimate the onset probability of each rare disease of this patient based on patient data that has been encrypted and subjected to the third preprocessing.

The third learning unit 327 uses the inspection data and the medical care data of the patient who has developed a rare disease and the inspection data and the medical care data of the patient who has developed a disease other than a rare disease after the third preprocessing as learning data, and causes the third estimation model 326 to learn a relationship between the inspection data and the medical care data of the patient and the onset probability of the rare disease of the patient.

The third learning unit 327 inputs inspection data and medical care data (excluding rare disease information), which are learning data after the third preprocessing, to the third estimation model 326, and performs third parameter update processing of updating a parameter of the third estimation model 326 so that the onset probability of each rare disease estimated by the third estimation model 326 approaches the rare disease actually diagnosed.

### [Learning processing]

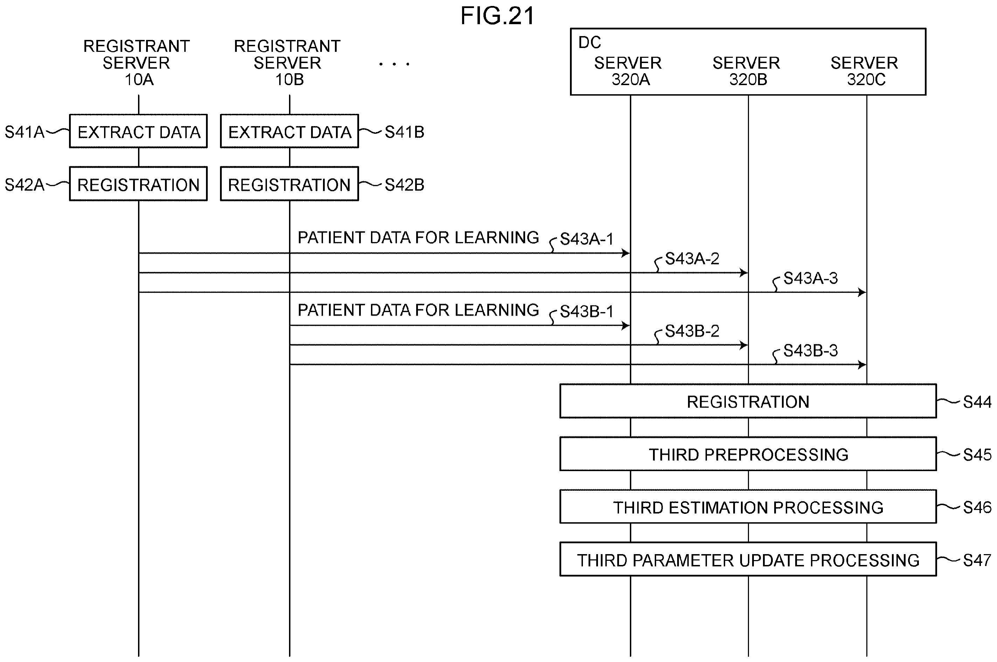

Next, learning processing according to the third embodiment will be described. FIG. 21 is a sequence diagram illustrating a processing procedure of learning processing according to the third embodiment.

Steps S41A to S44 illustrated in FIG. 21 are the same processing as steps S1A to S4 illustrated in FIG. 11.

After performing the third preprocessing on the patient data for learning (step S45), the servers 320A to 320C perform the third estimation processing of estimating the onset probability of this patient for each of the plurality of rare diseases based on the patient data for learning after the third preprocessing (step S46).

The servers 320A to 320C perform the third parameter update processing of updating the parameters of the third estimation model 326 so that the onset probability of each rare disease estimated by the third estimation model 326 approaches the rare disease actually diagnosed (step S47). The servers 320A to 320C repeatedly execute steps S45 to S47 until a predetermined end condition is satisfied. The servers 320A to 320C execute steps S44 to S47 on the secret calculation AI.

### (Estimation processing)

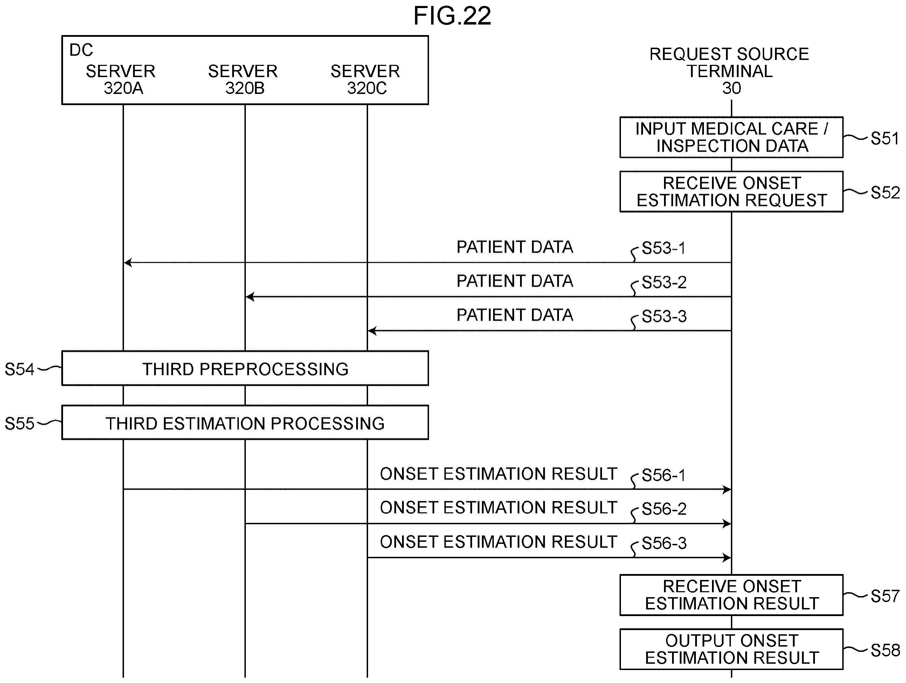

Next, estimation processing according to the third embodiment will be described. FIG. 22 is a sequence diagram illustrating a processing procedure of estimation processing according to the third embodiment.

Steps S51 to S53-3 illustrated in FIG. 22 are the same processing as steps S11 to S13-3 illustrated in FIG. 12.

After performing the third preprocessing on the estimation target patient data (step S54), the servers 320A to 320C perform the third estimation processing of estimating the onset probability of this patient for each of the plurality of rare diseases based on the estimation target patient data after the third preprocessing (step S55). The servers 320A to 320C execute steps S54 and S55 on the secret calculation AI. The servers 320A to 320C transmit the estimation result of the onset probability of a rare disease of the estimation target patient whose disease name cannot be determined to the request source terminal 30 (steps S56-1 to S56-3). Steps S57 and S58 illustrated in FIG. 22 are the same processing as steps S17 and S18 illustrated in FIG. 12.

### (Effect of third embodiment)

In the third embodiment, since the dimension of the middle item can be further reduced by the third preprocessing, it is possible to improve the learning time of the third estimation model 326 and reduce the amount of data requested for learning, and even if all the middle items are unknown data and the like, the input is not sparse, so that the learning of the third estimation model 326 can be appropriately executed.

### (Fourth embodiment)

Next, a fourth embodiment will be described. The DC server according to the fourth embodiment includes each estimation unit and each learning unit of the first to third embodiments, evaluates the estimation accuracy of each estimation model, and sets the estimation result of which estimation model is adopted based on the evaluation. In addition, the DC server may set which estimation model is intensively learned based on the evaluation result of the estimation accuracy of each estimation model.

The processing system according to the fourth embodiment includes a server 420 instead of the server 20 according to the first embodiment. Note that, also in the fourth embodiment, the DC includes a plurality of servers 420 (420A to 420C to be described later), and the plurality of servers 420 executes various types of processing to be described below by distributing and storing various types of data in a state of being fragmented into shares and performing multi-party calculation among the plurality of servers 420.

### (Server)

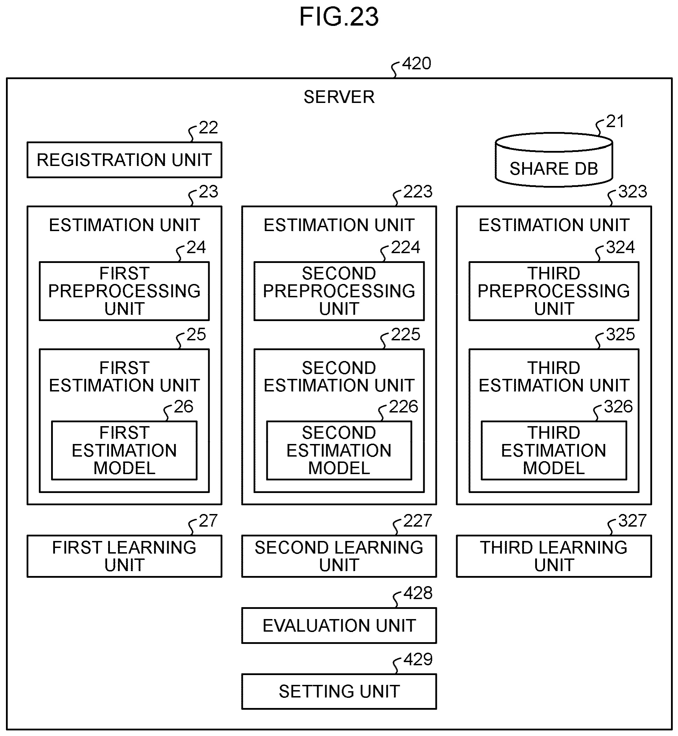

Next, a configuration of the server 420 will be described. FIG. 23 is a diagram schematically illustrating an example of a configuration of the server 420 according to the fourth embodiment. As compared with 20 illustrated in FIG. **3****,** the server 420 includes the estimation unit 223 and the second learning unit 227 of the server 220 illustrated in FIG. 13, the estimation unit 323 and the third learning unit 327 illustrated in FIG. 18, an evaluation unit 428, and a setting unit 429.

In the estimation phase, the evaluation unit 428 evaluates the estimation accuracy of the first estimation unit 25, the second estimation unit 225, and the third estimation unit 325 based on the estimation result by the first estimation unit 25, the estimation result by the second estimation unit 225, and the estimation result by the third estimation unit 325. For example, the evaluation unit 428 evaluates the estimation accuracy of each estimation unit by comparing the estimation probabilities of the first estimation unit 25, the second estimation unit 225, and the third estimation unit 325 for arbitrary patient data with the rare disease developed by the arbitrary patient.

The setting unit 429 sets which one of the estimation results of the first estimation unit 25, the second estimation unit 225, and the third estimation unit 325 is adopted based on the evaluation result by the evaluation unit 428.

A case where the second estimation unit 225 has the highest estimation accuracy and the third estimation unit 325 and the first estimation unit 25 have lower estimation accuracy in this order will be described as an example. In this case, for example, the setting unit 429 adopts only the estimation result of the second estimation unit 225 having the highest accuracy and transmits the estimation result to the request source terminal 30. Alternatively, the setting unit 429 may cause only the estimation unit 223 to execute preprocessing and estimation processing. In addition, the setting unit 429 sets a weight according to the level of estimation accuracy for each estimation unit, and transmits the weighted sum of the estimation results of the first estimation unit 25, the second estimation unit 225, and the third estimation unit 325 to the request source terminal 30.

In addition, the setting unit 429 may set learning processing. For example, in a case where it is difficult to learn all the estimation models due to resources of the server 420 and the like, the setting unit 429 may cause the second learning unit 227 to execute learning by giving priority to learning for the second estimation model 226 of the second estimation unit 225 having the highest accuracy.

### [Learning processing]

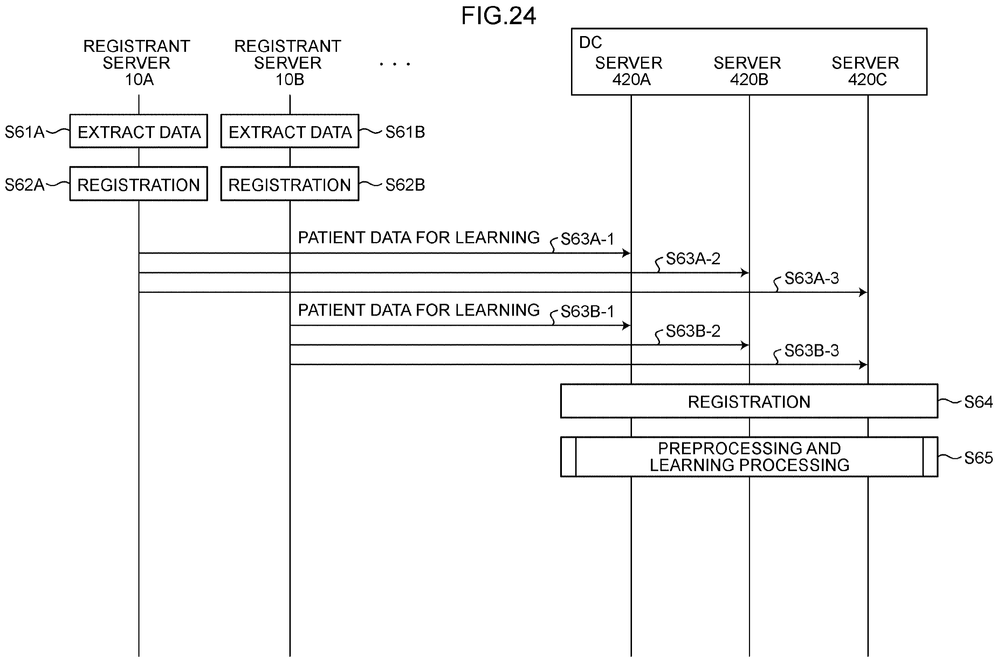

Next, learning processing according to the fourth embodiment will be described. FIG. 24 is a sequence diagram illustrating a processing procedure of learning processing according to the fourth embodiment.

Steps S61A to S64 illustrated in FIG. 24 are the same processing as steps S1A to S4 illustrated in FIG. 11. The servers 420A to 420C execute preprocessing and learning processing based on the patient data for learning (step S65).

### [Processing procedure of preprocessing and learning processing]

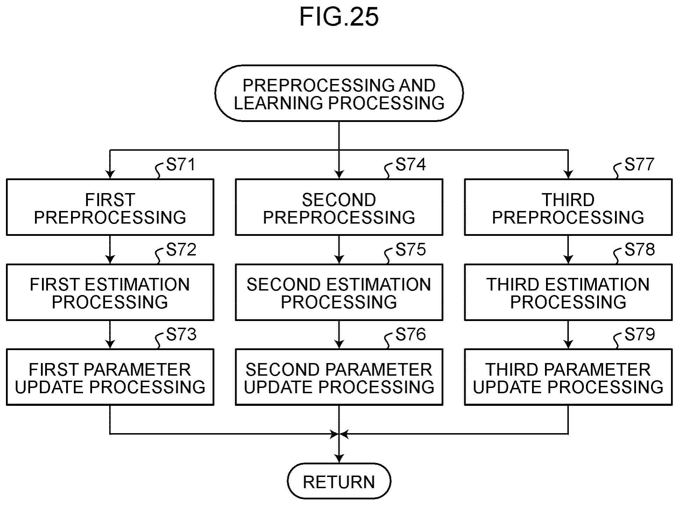

Next, preprocessing and learning processing (step S65) illustrated in FIG. 24 will be described. FIG. 25 is a sequence diagram illustrating a processing procedure of preprocessing and learning processing illustrated in FIG. 24.

Each estimation unit 23 and each first learning unit 27 of the servers 420A to 420C perform the same processing as steps S5 to S7 illustrated in FIG. 11 to perform first preprocessing (step S71), first estimation processing (step S72), and first parameter update processing (step S73).

Each estimation unit 223 and each second learning unit 227 of the servers 420A to 420C perform the same processing as steps S25 to S27 illustrated in FIG. 16 to perform second preprocessing (step S74), second estimation processing (step S75), and second parameter update processing (step S76).

Each estimation unit 323 and each third learning unit 327 of the servers 420A to 420C perform the same processing as steps S45 to S47 illustrated in FIG. 21 to perform third preprocessing (step S77), third estimation processing (step S78), and third parameter update processing (step S79).

Steps S71 to S73, steps S74 to S76, and steps S77 to S79 may not be parallel processing. In addition, any of steps S71 to S73, steps S74 to S76, and steps S77 to S79 may be executed according to the setting of the setting unit 429.

### (Estimation processing)

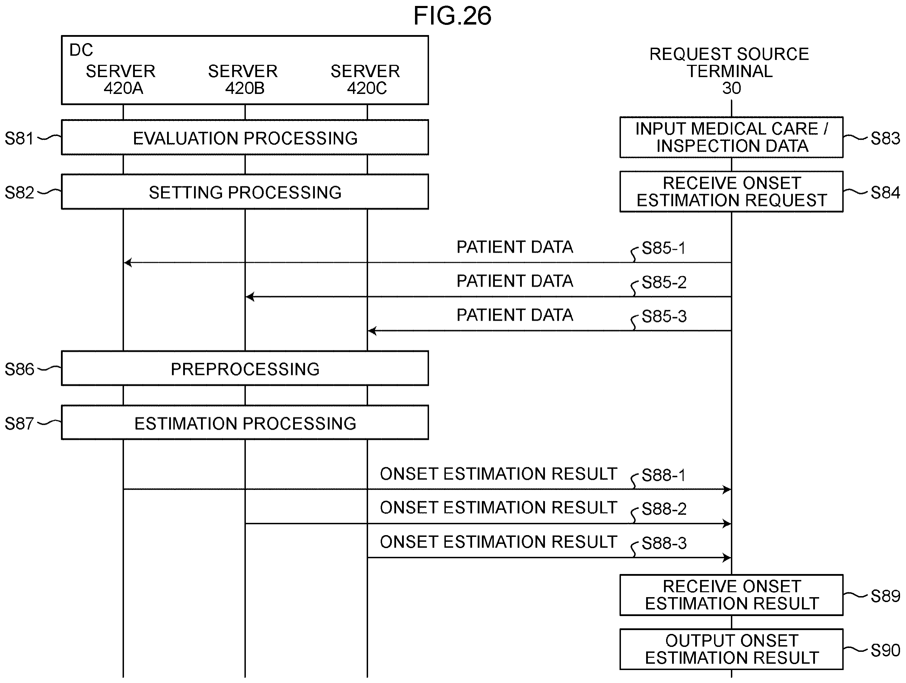

Next, estimation processing according to the fourth embodiment will be described. FIG. 26 is a sequence diagram illustrating a processing procedure of estimation processing according to the fourth embodiment.

The servers 420A to 420C perform evaluation processing of evaluating the estimation accuracy of the first estimation unit 25, the second estimation unit 225, and the third estimation unit 325 based on the estimation result by the first estimation unit 25, the estimation result by the second estimation unit 225, and the estimation result by the third estimation unit 325 (step S81).

The setting unit 429 performs setting processing of setting which one of the estimation results of the first estimation unit 25, the second estimation unit 225, and the third estimation unit 325 is adopted based on the evaluation result by the evaluation unit 428 (step S82). Steps S81 and S82 are periodically executed, for example. Alternatively, steps S81 and S82 are executed at predetermined timing such as a case where the data accumulation amount exceeds a predetermined amount, a case where the number of times of estimation exceeds a predetermined number of times, and a case where the estimation accuracy of the employed estimation model is lower than the target accuracy.

Steps S83 to S85-3 illustrated in FIG. 26 are the same processing as steps S11 to S13-3 illustrated in FIG. 12.

The servers 420A to 420C cause the estimation units 23, 223, and 323 to execute preprocessing (step S86) and estimation processing (step S87), respectively, adopt the estimation result of the estimation unit whose adoption is set in the setting processing, and transmit the estimation result to the request source terminal 30 (steps S88-1 to S88-3). Note that the servers 420A to 420C may cause only the estimation units 23, 223, and 323 whose adoption has been set in the setting processing to execute the preprocessing and the estimation processing, or may transmit the weighted sum of the estimation results of the first estimation unit 25, the second estimation unit 225, and the third estimation unit 325 to the request source terminal 30.

Steps S89 and S90 illustrated in FIG. 26 are the same processing as steps S17 and S18 illustrated in FIG. 12.

### (Effect of fourth embodiment)

As described above, the servers 420A to 420C according to the fourth embodiment include the estimation units and the learning units of the first to third embodiments, evaluate the estimation accuracy of each estimation model, and set the estimation result of which estimation model is adopted based on the evaluation, so that it is possible to provide the onset estimation result to the request source terminal 30 with stable accuracy. In addition, since the servers 420A to 420C set which estimation model is mainly learned based on the evaluation result of the estimation accuracy of each estimation model, it is possible to efficiently learn or re-learn the estimation model.

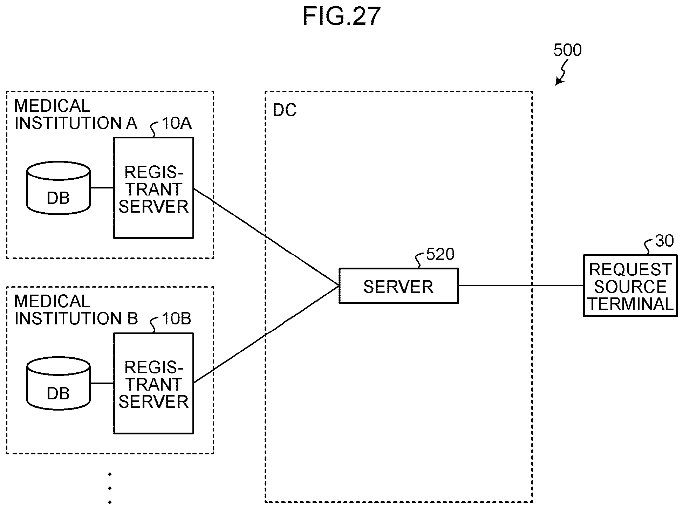

Note that, in the first to fourth embodiments, the case where the learning processing and the estimation processing are executed on the secret calculation AI has been described, but the present invention is not limited thereto. FIG. 27 is a block diagram illustrating another example of the configuration of the processing system according to the first embodiment.

As illustrated in a processing system 500 of FIG. 27, the data distributed and stored among the servers 20A, 20B, and 20C may be stored only in a server 520. In addition, the learning processing and the estimation processing performed by the servers 20A, 20B, and 20C performing the multi-party calculation may be executed only by the server 520. That is, the learning processing and the estimation processing according to first to fourth embodiments may be executed without being encrypted.

### (System configuration and the like)

In addition, each component of each device illustrated in the drawings is functionally conceptual, and is not necessarily physically configured as illustrated in the drawings. That is, a specific form of distribution and integration of each device is not limited to the illustrated form, and all or a part thereof can be functionally or physically distributed and integrated in an arbitrary unit according to various loads, usage conditions, and the like. Furthermore, all or an arbitrary part of each processing function performed in each device can be realized by a central processing unit (CPU), a graphics processing unit (GPU), and a program analyzed and executed by the CPU or the GPU, or can be realized as hardware by wired logic.

Among the processes described in the present embodiment, all or part of the processing described as being performed automatically can be performed manually, or all or part of the processing described as being performed manually can be performed automatically by a known method. In addition, the processing procedure, the control procedure, the specific name, and the information including various data and parameters illustrated in the document and the drawings can be arbitrarily changed unless otherwise specified.

### [Program]

In addition, it is also possible to create a program in which the processing executed by the registrant servers 10A and 10B, the servers 20, 220, 320, 420, and 520, and the request source terminal 30 explained in the above embodiments are described in a language executable by a computer. For example, it is also possible to create a program in which the processing executed by the registrant servers 10A and 10B, the servers 20, 220, 320, 420, and 520, and the request source terminal 30 in the above embodiments are described in a language executable by a computer. In this case, when the computer executes the program, the same effects as those of the above embodiment can be obtained. Further, the program may be recorded in a computer-readable recording medium, and the program recorded in the recording medium may be read and executed by a computer to execute processing similar to the above-described embodiment.

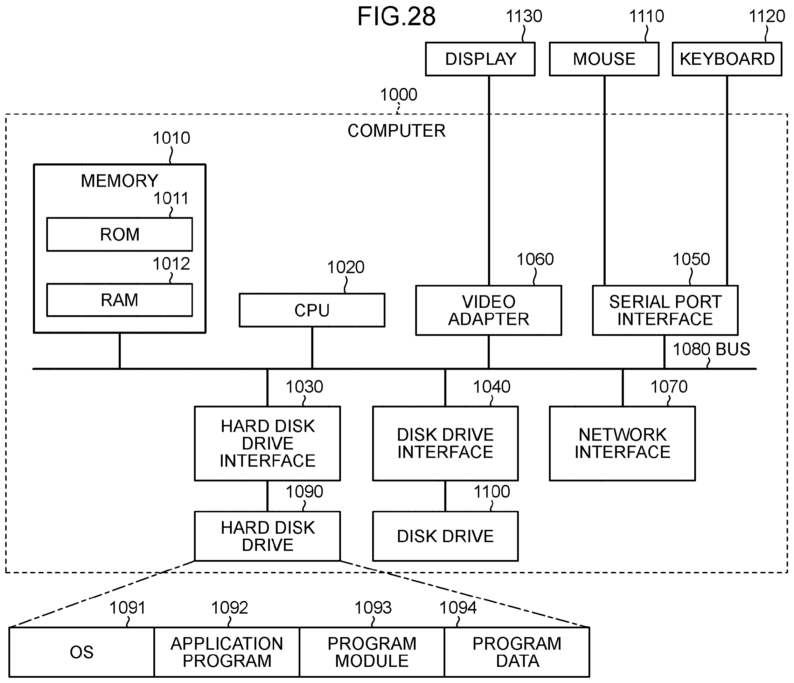

FIG. 28 is a diagram illustrating a computer that executes a program. As illustrated in FIG. 28, the computer 1000 includes, for example, a memory 1010, a CPU 1020, a hard disk drive interface 1030, a disk drive interface 1040, a serial port interface 1050, a video adapter 1060, and a network interface 1070, and these units are connected by a bus 1080.

As illustrated in FIG. 28, the memory 1010 includes a read only memory (ROM) 1011 and a random access memory (RAM) 1012. The ROM 1011 stores, for example, a boot program such as a basic input output system (BIOS). The hard disk drive interface 1030 is connected to a hard disk drive 1090 as illustrated in FIG. 28. The disk drive interface 1040 is connected to a disk drive 1100. For example, a removable storage medium such as a magnetic disk or an optical disk is inserted into the disk drive 1100. The serial port interface 1050 is connected to, for example, a mouse 1110 and a keyboard 1120. The video adapter 1060 is connected to, for example, a display 1130.

Here, as illustrated in FIG. 28, the hard disk drive 1090 stores, for example, an operating system (OS) 1091, an application program 1092, a program module 1093, and program data 1094. That is, the program described above is stored, for example, in the hard disk drive 1090 as a program module in which a command executed by the computer 1000 is described.

In addition, the various data described in the above embodiment are stored in, for example, the memory 1010 or the hard disk drive 1090 as program data. Then, the CPU 1020 reads the program module 1093 and the program data 1094 stored in the memory 1010 and the hard disk drive 1090 into the RAM 1012 as necessary, and executes various processing procedures.

Note that the program module 1093 and the program data 1094 related to the program are not limited to being stored in the hard disk drive 1090, and may be stored in, for example, a detachable storage medium and read by the CPU 1020 via a disk drive and the like. Alternatively, the program module 1093 and the program data 1094 related to the program may be stored in another computer connected via a network (local area network (LAN), wide area network (WAN), and the like) and read by the CPU 1020 via the network interface 1070.

The above-described embodiments and modifications thereof are included in the invention described in the claims and the equivalent scope thereof as well as included in the technology disclosed in the present application.

### [Explanation of Reference]

10A, 10B Registrant server
11 DATA EXTRACTION UNIT
12 REGISTRATION UNIT
20, 20A to 20C, 220, 220A to 220C, 320, 320A to 320C, 420, 420A to 420C, 520 Server
21 SHARE DB
22 REGISTRATION UNIT
23, 223, 323 Estimation unit
24 FIRST PREPROCESSING UNIT
25 FIRST ESTIMATION UNIT
26 FIRST ESTIMATION MODEL
27 FIRST LEARNING UNIT
30 REQUEST SOURCE TERMINAL
31 RECEPTION UNIT
32 ESTIMATION RESULT RECEPTION UNIT
33 ESTIMATION RESULT OUTPUT UNIT
100, 500 Processing system
224 SECOND PREPROCESSING UNIT
225 SECOND ESTIMATION UNIT
226 SECOND ESTIMATION MODEL
227 SECOND LEARNING UNIT
324 THIRD PREPROCESSING UNIT
325 THIRD ESTIMATION UNIT
326 THIRD ESTIMATION MODEL
327 THIRD LEARNING UNIT
428 EVALUATION UNIT
429 SETTING UNIT

## Claims

1. A learning device comprising:
a registration unit that registers at least inspection data and medical care data of a patient who has developed a rare disease from a plurality of medical institutions;
a preprocessing unit that performs predetermined preprocessing on inspection data and medical care data of a patient;
an estimation unit that estimates an onset probability of an estimation target patient for each of a plurality of rare diseases based on the inspection data and medical care data of the estimation target patient after the preprocessing by using an estimation model that estimates an onset probability for each of the plurality of rare diseases; and
a learning unit that uses at least the inspection data and the medical care data of the patient who has developed a rare disease after the preprocessing as learning data, and causes the estimation model to learn a relationship between the inspection data and the medical care data of the patient and an onset probability of a rare disease of the patient.

2. The learning device according to claim 1, wherein the preprocessing unit performs, for the inspection data and the medical care data, first preprocessing of setting items related to a patient background, a medical history, a clinical examination finding, or an inspection finding in stages according to granularity, and converting data of each item into corresponding categorical variables.

3. The learning device according to claim 1, wherein the preprocessing unit performs, for the inspection data and the medical care data, second preprocessing of setting items related to a patient background, a medical history, a clinical examination finding, or an inspection finding in stages according to granularity, converting data of each item into corresponding categorical variables, labeling a categorical variable part of an item with a finest granularity according to a meaning of data corresponding to the categorical variables, and compressing a same label.

4. The learning device according to claim **1,** wherein the preprocessing unit performs, for the inspection data and the medical care data, third preprocessing of setting items related to a patient background, a medical history, a clinical examination finding, or an inspection finding in stages, converting data of each item into corresponding categorical variables, labeling a categorical variable part of a first item with a finest granularity according to a meaning of data corresponding to the categorical variables, compressing a same label, and expressing a second item having coarser granularity than the first item by a number of counts of each compressed label of the first item belonging to the second item.

5. The learning device according to claim 1, wherein
the preprocessing unit includes
a first preprocessing unit that performs, for the inspection data and the medical care data, first preprocessing of setting items related to a patient background, a medical history, a clinical examination finding, or an inspection finding in stages according to granularity, and converting data of each item into corresponding categorical variables,
a second preprocessing unit that performs, for the inspection data and the medical care data, second preprocessing of setting items related to a patient background, a medical history, a clinical examination finding, or an inspection finding in stages according to granularity, converting data of each item into corresponding categorical variables, labeling a categorical variable part of an item with a finest granularity according to a meaning of data corresponding to the categorical variables, and compressing a same label, and
a third preprocessing unit that performs, for the inspection data and the medical care data, third preprocessing of setting items related to a patient background, a medical history, a clinical examination finding, or an inspection finding in stages, converting data of each item into corresponding categorical variables, labeling a categorical variable part of a first item with a finest granularity according to a meaning of data corresponding to the categorical variables, compressing a same label, and expressing a second item having coarser granularity than the first item by a number of counts of each compressed label of the first item belonging to the second item, wherein
the estimation unit includes
a first estimation unit including a first estimation model that estimates an onset probability of the estimation target patient for each of the plurality of rare diseases based on the inspection data and medical care data of the patient after the first preprocessing,
a second estimation unit including a second estimation model that estimates an onset probability of the estimation target patient for each of the plurality of rare diseases based on the inspection data and medical care data of the patient after the second preprocessing, and
a third estimation unit including a third estimation model that estimates an onset probability of the estimation target patient for each of the plurality of rare diseases based on the inspection data and medical care data of the patient after the third preprocessing, and
the learning unit includes
a first learning unit that uses at least the inspection data and the medical care data of the patient who has developed a rare disease after the first preprocessing as learning data, and causes the first estimation model to learn a relationship between the inspection data and the medical care data of the patient and an onset probability of a rare disease of the patient,
a second learning unit that uses at least the inspection data and the medical care data of the patient who has developed a rare disease after the second preprocessing as learning data, and causes the second estimation model to learn a relationship between the inspection data and the medical care data of the patient and an onset probability of a rare disease of the patient, and
a third learning unit that uses at least the inspection data and the medical care data of the patient who has developed a rare disease after the third preprocessing as learning data, and causes the third estimation model to learn a relationship between the inspection data and the medical care data of the patient and an onset probability of a rare disease of the patient.

6. The learning device according to claim 1, wherein the learning device distributes and stores data in a plurality of servers in a state of a fragmented share and is realized by secret calculation artificial intelligence (AI) in which the plurality of servers performs calculation processing on secret calculation.

7. A learning method executed by a learning device, the learning method comprising:
a process of registering at least inspection data and medical care data of a patient who has developed a rare disease from a plurality of medical institutions;
a process of performing predetermined preprocessing on inspection data and medical care data of a patient;
a process of estimating an onset probability of an estimation target patient for each of a plurality of rare diseases based on the inspection data and medical care data of the estimation target patient after the preprocessing by using an estimation model that estimates an onset probability for each of the plurality of rare diseases; and
a process of using at least the inspection data and the medical care data of the patient who has developed a rare disease after the preprocessing as learning data, and causing the estimation model to learn a relationship between the inspection data and the medical care data of the patient and an onset probability of a rare disease of the patient.

8. A learning program comprising:
a step of registering at least inspection data and medical care data of a patient who has developed a rare disease from a plurality of medical institutions;
a step of performing predetermined preprocessing on inspection data and medical care data of a patient;
a step of estimating an onset probability of an estimation target patient for each of a plurality of rare diseases based on the inspection data and medical care data of the estimation target patient after the preprocessing by using an estimation model that estimates an onset probability for each of the plurality of rare diseases; and
a step of using at least the inspection data and the medical care data of the patient who has developed a rare disease after the preprocessing as learning data, and causing the estimation model to learn a relationship between the inspection data and the medical care data of the patient and an onset probability of a rare disease of the patient.

9. An estimation device comprising:
a preprocessing unit that performs predetermined preprocessing on inspection data and medical care data of a patient; and
an estimation unit that estimates an onset probability of an estimation target patient for each of a plurality of rare diseases based on inspection data and medical care data of an estimation target patient after the preprocessing using an estimation model that uses at least inspection data and medical care data of a patient who has developed a rare disease after the preprocessing as learning data and learns a relationship between inspection data and medical care data of a patient and an onset probability of a rare disease of the patient, the estimation model estimating an onset probability for each of the plurality of rare diseases.

10. The estimation device according to claim **9,** wherein the preprocessing unit performs, for the inspection data and the medical care data, first preprocessing of setting items related to a clinical examination background, a medical history, a clinical examination finding, or an inspection finding in stages according to granularity, and converting data of each item into corresponding categorical variables.

11. The estimation device according to claim **9,** wherein the preprocessing unit performs, for the inspection data and the medical care data, second preprocessing of setting items related to a clinical examination background, a medical history, a clinical examination finding, or an inspection finding in stages according to granularity, converting data of each item into corresponding categorical variables, labeling a categorical variable part of an item with a finest granularity according to a meaning of data corresponding to the categorical variables, and compressing a same label.

12. The estimation device according to claim **9,** wherein the preprocessing unit performs, for the inspection data and the medical care data, third preprocessing of setting items related to a clinical examination background, a medical history, a clinical examination finding, or an inspection finding in stages, converting data of each item into corresponding categorical variables, labeling a categorical variable part of a first item with a finest granularity according to a meaning of data corresponding to the categorical variables, compressing a same label, and expressing a second item having coarser granularity than the first item by a number of counts of each compressed label of the first item belonging to the second item.

13. The estimation device according to claim 9, wherein
the preprocessing unit includes
a first preprocessing unit that performs, for the inspection data and the medical care data, first preprocessing of setting items related to a clinical examination background, a medical history, a clinical examination finding, or an inspection finding in stages according to granularity, and converting data of each item into corresponding categorical variables,
a second preprocessing unit that performs, for the inspection data and the medical care data, second preprocessing of setting items related to a clinical examination background, a medical history, a clinical examination finding, or an inspection finding in stages according to granularity, converting data of each item into corresponding categorical variables, labeling a categorical variable part of an item with a finest granularity according to a meaning of data corresponding to the categorical variables, and compressing a same label, and
a third preprocessing unit that performs, for the inspection data and the medical care data, third preprocessing of setting items related to a clinical examination background, a medical history, a clinical examination finding, or an inspection finding in stages, converting data of each item into corresponding categorical variables, labeling a categorical variable part of a first item with a finest granularity according to a meaning of data corresponding to the categorical variables, compressing a same label, and expressing a second item having coarser granularity than the first item by a number of counts of each compressed label of the first item belonging to the second item, wherein
the estimation unit includes
a first estimation unit that estimates an onset probability of the estimation target patient based on the inspection data and medical care data of the patient after the first preprocessing using a first estimation model that has learned the inspection data and the medical care data of a patient who has developed a rare disease,
a second estimation unit that estimates an onset probability of the estimation target patient based on the inspection data and medical care data of the patient after the second preprocessing using a second estimation model that has learned the inspection data and the medical care data of a patient who has developed a rare disease, and
a third estimation unit that estimates an onset probability of the estimation target patient based on the inspection data and medical care data of the patient after the third preprocessing using a third estimation model that has learned the inspection data and the medical care data of a patient who has developed a rare disease.

14. The estimation device according to claim 13, including:
an evaluation unit that evaluates estimation accuracy of the first estimation unit, the second estimation unit, and the third estimation unit based on an estimation result by the first estimation unit, an estimation result by the second estimation unit, and an estimation result by the third estimation unit, and
a setting unit that sets which estimation result of the first estimation unit, the second estimation unit, and/or the third estimation unit is adopted based on an evaluation result by the evaluation unit.

15. The estimation device according to claim 9, wherein the estimation device distributes and stores data in a plurality of servers in a state of a fragmented share and is realized by secret calculation artificial intelligence (AI) in which the plurality of servers performs calculation processing on secret calculation.

16. An estimation method executed by an estimation device, the estimation method comprising:
a process of performing predetermined preprocessing on inspection data and medical care data of a patient; and
a process of estimating an onset probability of an estimation target patient for each of a plurality of rare diseases based on inspection data and medical care data of an estimation target patient after the preprocessing using an estimation model that uses at least inspection data and medical care data of a patient who has developed a rare disease after the preprocessing as learning data and learns a relationship between inspection data and medical care data of a patient and an onset probability of a rare disease of the patient, the estimation model estimating an onset probability for each of the plurality of rare diseases.

17. An estimation program comprising:
a step of performing predetermined preprocessing on inspection data and medical care data of a patient; and
a step of estimating an onset probability of an estimation target patient for each of a plurality of rare diseases based on inspection data and medical care data of an estimation target patient after the preprocessing using an estimation model that uses at least inspection data and medical care data of a patient who has developed a rare disease after the preprocessing as learning data and learns a relationship between inspection data and medical care data of a patient and an onset probability of a rare disease of the patient, the estimation model estimating an onset probability for each of the plurality of rare diseases.
